# EUROPÄISCHE PATENTANMELDUNG

(11) **EP 2 706 044 A1**
(43) Veröffentlichungstag der Anmeldung: **12.03.2014**
(21) Anmeldenummer: 12183547.4
(22) Anmeldetag: 07.09.2012
(51) Int. Cl.: C02F 3/00, C02F 3/28, C02F 9/00

(54) **Behandlung von Abwässern, insbesondere von sulfat- und/oder schwermetallhaltigen Minenwässern**

(71) Anmelder: Siemens Aktiengesellschaft, 80333 München (DE)
(72) Erfinder: Klein, Robert, 01069 Dresden (DE); Schlömann, Michael, Prof., 09599 Freiberg (DE); Mühling, Martin, Dr., 09599 Freiberg (DE); Glombitza, Franz, Dr., 09224 Chemnitz (DE); Janneck, Eberhard, Dr., 09599 Freiberg (DE); Otto, Matthias, Prof., 09600 Oberschöna (DE); Rathsack, Philipp, 09599 Freiberg (DE)

(57) **Zusammenfassung**

Die Erfindung betrifft eine Vorrichtung sowie ein Verfahren zur Behandlung von Abwässern, insbesondere von sulfat- und/oder schwermetallhaltigen Minenwässern.

Die Vorrichtung weist einen Reaktorraum (2), eine im Wesentlichen in dem Reaktorraum (2) angeordnete Umwälzvorrichtung (60) zu einer Umwälzung eines Umwälzstroms (46) aus einem sich in dem Reaktorraum (2) befindenden Abwasser (10) sowie einem in dem Reaktorraum (2) angeordneten Trägermaterial (41), auf welchem Biomasse (45) eines Biofilms (44) immobilisiert ist, und eine in dem Reaktorraum (2) angeordnete, von dem Umwälzstrom (46) durchströmbare Abscheidvorrichtung (20), welche eine Filtervorrichtung (25) aufweist, welche an das Trägermaterial (41) angepasste Öffnungen (29) aufweist, auf, wobei aus dem Umwälzstrom (46) die während der Umwälzung des Umwälzstroms (46) von dem Trägermaterial (42) gelöste Biomasse (45) aus dem Umwälzstrom (46) ausscheidbar ist.

Nach dem Verfahren wird der Umwälzstrom (48) unter Verwendung der Umwälzvorrichtung (60) in dem Reaktorraum (2) umgewälzt, wobei bei der Umwälzung die auf dem Trägermaterial (41) immobolisierte Biomasse (45) von dem Trägermaterial (41) gelöst und die gelöste Biomasse (45) aus dem Abwasser (10) abgeschieden (132) wird. Über die Umwälzung (130) wird der Biofilm (44), insbesondere ein Anwachsen der auf dem Trägermaterial (41) immobilisierten Biomasse (45) des Biofilms (44) und/oder eine Dicke des Biofilms (44), kontrolliert (140).

## Beschreibung

Die Erfindung betrifft eine Vorrichtung sowie ein Verfahren zur Behandlung von Abwässern, insbesondere von sulfat- und/oder schwermetallhaltigen Minenwässern.

Im Bergbau fallen in unterschiedlichsten Aufbereitungsverfahren, wie beispielsweise beim Mahlen oder Flotieren des abgebauten Gesteins, oder bei der Veredelung des Materials verschiedenste Prozess- und Aufbereitungswässer an. Diese enthalten neben Gangart auch Chemikalien, welche bei der Aufbereitung zugesetzt werden, und werden derzeit in großen Mengen in Speichereinrichtungen geleitet.

Mit der Zeit sinkt aufgrund fortschreitender chemischer Reaktionen der pH-Wert in den gespeicherten Prozess- und Aufbereitungswässern ab und Metallionen, welche toxisch sein können, werden aus der Gangart herausgelöst, die sich in der Flüssigphase anreichern.

Weiterhin ist ein Abwasserstrom zu berücksichtigen, der sich zusammensetzt aus einerseits Abwässern aus aktiven und bereits aufgegebenen Tief- und Tagebaugruben und andererseits Sickerwasser aus Aufbereitungsrückständen, Abraum und anderen mineralischen Abfallmengen. Dieser Abwasserstrom ist durch einen niedrigen pH-Wert und einen hohen Sulfatgehalt gekennzeichnet und enthält gelöste Metalle unterschiedlicher Art.

Daher wird dieser Abwasserstrom auch als "saurer Minenablauf" (acid mine drainage AMD) oder "saurer Gesteinsablauf" (acid rock drainage ARD) bezeichnet.

Er entsteht durch Oxidation sulfidischer Minerale aufgrund einer Infiltration mit Oberflächenwasser, Grundwasser und Sauerstoff aus der Luft, wie auch einer Aktivität von Sulfidoxidierenden Bakterien der Gattung Acidithiobactillus ferro-+oxidans und Acidithiobactillus thiooxidans.

Die Prozess- und Aufbereitungswässer vor und während der Speicherung sowie der Abwasserstrom werden, einzeln oder auch in Mischung miteinander, nachfolgend vereinfachend als Minenwässer bezeichnet. Auch mitumfasst als Minenwässer sollen auch mit anderen Verfahren, beispielsweise einer Membranfiltration, aufkonzentrierte Minenwässer sein.

Aufgrund stetig steigender Preise für Frischwasser und einem wachsenden Bewusstsein für das Umweltrisiko, das derartige Minenwässer z.B. im Hinblick auf eine Verunreinigung von Oberflächen- und Grundwasser darstellen, gewinnt das Thema Aufbereitung von Minenwässern und Nutzung als Frisch- und/oder Brauchwasserquelle ständig an Bedeutung.

Es sind bereits diverse Aktiv- und Passivtechnologien bekannt, um sulfat- und/oder (schwer-)metallhaltige Abwässer bzw. Minenwässer zu behandeln bzw. zu reinigen bzw. aus diesen das Sulfat und die (Schwer-)Metalle auszufällen, beispielsweise eine chemische Fällung unter Verwendung von Kalkwasser, Ionenaustauschprozesse und Membranverfahren/- filtration, insbesondere Nanofiltration.

Bei einer Membranfiltration, insbesondere einer Nanofiltration oder Umkehrosmose, handelt es sich um Filterprozesse, die unter Druck stattfinden, wobei ein Aufgabestrom an Minenwässern in zwei Teilströme aufgespalten wird.

Bei dem ersten gebildeten Teilstrom handelt es sich um das sogenannte Permeat, hier in Form eines überwiegenden Teils des Wassers aus dem Aufgabestrom, das durch die semipermeable Membran gelangt. Das Permeat bildet ein niedrig mineralisiertes Prozesswasser mit saurem pH-Wert, beispielsweise im Bereich von üblicherweise etwa pH = 4 bis 5. Es kann in dieser Form - gegebenenfalls unter Berücksichtigung gültiger Gesetzgebung bzw. Vorschriften, wie insbesondere lokale Abwasservorschriften, - in die Umwelt abgegeben werden, beispielsweise in ein fließendes Gewässer oder Leitungsabwasser.

Das Permeat wird aber bevorzugt neutralisiert und/oder mit anderen Wässern, beispielsweise frischem Leitungswasser, gemischt und verdünnt, um es als Prozesswasser wiederzuverwenden.

Bei dem zweiten Teilstrom handelt es sich um das Konzentrat, das einen kleinen Rest des Wassers mit den im Aufgabestrom im Wesentlichen enthaltenen Verunreinigungen umfasst.

Aus der EP 1 147 063 B1 ist eine Behandlung von Minenwässern durch biologische Behandlung mittels biologischer Mikroorganismen, wie durch (Sulfat-/Metall-)reduzierende Bakterien, bekannt.

Die Reinigungswirkung bei dieser biologischen, auf Mikroorganismen bzw. (Sulfat-/Metall-)reduzierende Bakterien basierenden Abwasserbehandlung beruht auf einer biologischen Reduktion von Sulfat bzw. Metallen - mittels Mikroorganismen, welche typischerweise in Form eines Biofilms angesiedelt sind.

So erfolgt hier mittels solcher Mikroorganismen bzw. solcher (Sulfat-/Metall-)reduzierender Bakterien eine Sulfatreduzierung zu Sulfid und damit eine einhergehende Fällung von Metallsulfiden. Auch bewirkt diese biologische Behandlung mittels dieser Mikroorganismen eine biologische Reduktion der (Schwer-)Metalle zu einem geringeren Ladungszustand (Valenz), in welcher Form das Metall als ein Metallsulfid, Metallcarbonat, Metalloxid oder Hydroxid, Metallphosphat oder als das elementare Metall ausfällt.

Als geeignete Sulfat-reduzierende Bakterien sind solche der Spezies der Genera: Desulfotomaculum KT7 (thermophil), die Spezies Desulforolobus ambivalens, Acidianus infernus, Acidianus brierley, Stygiolobus azoricus (mesophil), Thermoproteus neutrophilus, Thermoproteus tenax, Thermodiscus maritimus (thermophil), Pyrobaculum islandicum, Pyrodictium occultum, Pyrodictium brockii (hyperthermophil) und andere Spezies der Genera Desulfovibrio, Desulfotomaculum, Desulfumonas, Thermodesulfobacterium, Desulfobulbus, Desulfobacter, Desulfococcus, Desulfonema, Desulfosarcina, und Desulfobacterium bekannt.

Als geeignete Bakterien für die biologische Reduktion von Schwermetallen bzw. Metallen, wie beispielsweise As, Mo, Fe, Cr, Mn, Se, Te, Sb, Bi, Hg, U, zu den geringeren Valenzen sind auch solche der Spezies der Genera Geobacter, Pseudomonas, Shewanella, Desulfovibrio, Desulfobacterium, Desulfomicrobium, Desulforomonas, Alteromonas genannt.

Es ist auch bekannt, organische Substanzen, wie Methanol, Ethanol und/oder Lactat (primäre Kohlenstoffquelle und Elektronendonor), zu oxidieren, um Elektronen zur Reduktion des Sulfats bereitzustellen.

Im Zuge einer Verstoffwechselung werden - u.a. auch von den vorherrschenden Milieubedingungen abhängig - intermediäre Metabolite gebildet, deren Art und Konzentration je nach Typ des Mikroorganismus variiert. Zu diesen Metaboliten gehören zum Beispiel Carbonsäure wie Essigsäure.

Die Gründe für deren Auftreten und extrazelluläre Konzentration sind vielfältig und komplex. Von Enterobakterien ist beispielsweise bekannt, dass bei Substratüberschuss keine vollständige Substratverwertung stattfindet und potentielle Energie durch Überproduktion von Metaboliten vergeudet wird.

Üblicherweise verwendet man für die biologische Abwasserbehandlung sogenannte Bio- bzw. Biofilmrektoren, in welchen die biologische und damit nachhaltige (Sulfat-/Metall-)Reduktion mittels der Mikroorganismen bzw. der (Sulfat-/Metall-)reduzierende Bakterien durchgeführt wird.

Dabei werden die Mikroorganismen bzw. die (Sulfat-/Metall-)reduzierenden Bakterien in Form eines Biofilms aus Biomasse auf einem Trägermaterial bzw. Aufwuchskörper, wie auf einem geeigneten, chemischen und physikalisch inerten, biologisch nicht abbaubaren Trägermaterial, beispielsweise einem natürlichen Trägermaterial, wie Sand oder (Kiesel-) Steine, oder auch Kunststoffträgermaterial, in einem Reaktorraum des Bioreaktors angesiedelt bzw. immobilisiert (Biobett), wodurch die Biomasse des Biofilms bei einem kontinuierlichen Betrieb des Bioreaktors vor einem Ausspülen aus dem Reaktorraum bzw. aus dem Bioreaktor geschützt wird und diese dadurch im Reaktionsraum des Bioreaktors bei höchstmöglicher Aktivität zurückgehalten wird. Das zu behandelnde Abwasser tritt - bei dessen Behandlung bzw. Reinigung - mit dem auf dem Trägermaterial immobilisierten Biofilm in Kontakt bzw. durchströmt diesen, wobei die biologische und damit nachhaltige (Sulfat-/Metall-)Reduktion stattfindet.

Verschiedene Formen von solchen Biofilmreaktoren zur Behandlung bzw. Reinigung von sulfat- und/oder (schwer-)metallhaltigen Minenwässern sind bekannt, beispielsweise Fest- oder Wirbelbettreaktoren.

Beim Einsatz von den Festbettreaktoren - hier befindet sich der Aufwuchskörper bzw. das Trägermaterial im Reaktorraum stationär in Ruhe (Festbett) - zur Sulfat- und/oder (Schwer-) Metallentfernung aus Minenwässern mit Hilfe der (mikro)biologischen (Sulfat-/Metall-)Reduktion kann es aber aufgrund der Ausfällung und Ablagerungen von Metallsulfiden im Reaktor zu einer Verblockung von offenen Poren des Aufwuchskörpers, hier eines Festbettes, und damit zur unerwünschten Ausbildung bevorzugter Fließwege kommen.

Dies führt zu einer sinkenden Kontaktzeit und -fläche der zu reinigenden Minenwässern mit dem auf dem Festbett wachsenden mikrobiellen Biofilm und einer daraus folgenden verringerten Reinigungseffizienz, also Sulfat- bzw. Metallentfernung, des Verfahrens.

Weiterhin überdecken auch die "biologisch" ausgefällten Metallsulfide den auf dem Festbett angewachsenen Biofilm, was den Stoffaustausch zwischen Abwasser und Biofilm deutlich verringert und ebenfalls die Reinigungsleistung negativ beeinflussen kann.

Auch eine mit der Zeit zu stark zunehmende Biofilmdicke - durch Anwachsen der Biomasse - kann zu einer Unterversorgung der untersten Biofilmschichten und damit zu deren Absterben führen. D.h., je größer die Dicke des Biofilm, desto schwieriger gelangen notwendige Nährstoffe in die untersten Schichten des Biofilms und können letztlich dort zu einem Absterben der Biomasse führen. Als Folge dessen können sich Teile des Biofilms bzw. Biomasse komplett vom Trägermaterial ablösen, was periodisch zu einem starken Rückgang der Reinigungsleistung des Festbettreaktors führen kann.

Bei solchen Festbettreaktoren ist demzufolge eine regelmäßige Reinigung gegebenenfalls auch eine Rückspülung des Trägermaterials über einen bestimmten Zeitraum notwendig, um die oben genannten Probleme zu minimieren/beseitigen. Während der Rückspülphase ist der eigentliche Reinigungsprozess bei den Minenwässern aber unterbrochen.

Als Alternative zu diesen Festbettreaktoren sind die Wirbelbettreaktoren bekannt.

Hier wird ein geringerer Anteil an Aufwuchskörper verwendet und dieser in dem Reaktorraum des Bioreaktors mittels Aufströmungen von Gas oder Flüssigkeiten in der "Schwebe" gehalten (Wirbelbett).

Somit kann eine Verstopfung im Trägermaterial und die dadurch resultierende Ausbildung bevorzugter Fließwege verhindert werden.

Nachteile dieser Wirbelbettreaktoren liegen aber im Energieaufwand, um die Aufwuchskörper in der Schwebe zu halten, sowie im geringeren Umsatz pro Reaktor-Volumen (Raum-Zeit Ausbeute), da das Trägermaterial weniger dicht im Reaktorraum gepackt ist.

Als weitere Alternative zu den Fest- und Wirbelbettreaktoren sind Bioreaktoren mit kontinuierlich/periodisch bewegtem Sandbett - als Aufwuchskörper für den (Sulfat-/Metall-)reduzierenden Biofilm - bekannt (Umwälzbett), beispielsweise aus der EP 1 147 063 B1.

In http://de.wikipedia.org/wiki/Eisenoxidierende Mikroorganismen (12.08.2012) ist eine biologische Eisenoxidation bei Minenwässern unter Verwendung von Mikroorganismen, hier Bakterien und Archaeen, beschrieben. Als Oxidationsmittel für die Eisenoxidation dient überwiegend Sauerstoff. Es ist hier weiter bekannt, dass die abiotische Oxidation von zweiwertigem Eisen (Fe(II)) zu dreiwertigem Eisen (Fe(III)) in einem hohen Maße vom pH-Wert und der Sauerstoffkonzentration abhängig ist.

Es ist weiter die Behandlung von Abwasser durch ein Ausstrippen von Gasen, wie von im Abwasser gelösten Schwefelwasserstoff, aus dem Abwasser bekannt. Das Ausstrippen verwendet eine Membran, die Gase passieren lässt und Flüssigkeiten zurückhält. Durch ein Partialdruckgefälle zwischen den beiden Membranseiten kann eine effektive Reduktion der Gase erreicht werden. Wie viel an gelöstem Gas, wie im Abwasser gelösten Schwefelwasserstoff (bezogen auf eine Gesamtsulfidfracht), aus dem Abwasser ausgestrippt werden kann, hängt auch vom vorhandenen pH-Wert des Abwassers ab.

In der DE 102 21 756 A1 ist eine biologische Oxidation von sauren, stark eisen- und sulfathaltigen Wässern, insbesondere Bergbauwässern, beschrieben.

Es ist Aufgabe der Erfindung, eine effiziente, industrielle, einfach und kostengünstig zu realisierende Aufarbeitung bzw. Behandlung von sulfat- und/oder schwermetallhaltigen Minenwässern aus Bergbaubetrieben bereitzustellen.

Die Aufgabe wird gelöst durch eine Vorrichtung sowie ein Verfahren zur Behandlung von Abwässern, insbesondere von sulfat- und/oder schwermetallhaltigen Minenwässern, mit den Merkmalen gemäß dem jeweiligen unabhängigen Patentanspruch.

Bevorzugte Weiterbildungen der Erfindung ergeben sich auch aus den abhängigen Ansprüchen. Alle Weiterbildungen beziehen sich sowohl auf die erfindungsgemäße Vorrichtung als auf das erfindungsgemäße Verfahren.

Die erfindungsgemäße Vorrichtung weist einen Reaktorraum sowie eine im Wesentlichen in dem Reaktorraum angeordnete Umwälzvorrichtung zu einer, insbesondere periodischen, Umwälzung eines Umwälzstroms aus einem sich in dem Reaktorraum befindenden Abwasser sowie einem in dem Reaktorraum angeordneten Trägermaterial, auf welchem Biomasse in Form eines Biofilms immobilisiert ist, auf.

Im Wesentlichen in dem Reaktorraum angeordnet meint dabei, dass auch Komponenten, Elemente und/oder Bauteile der erfindungsgemäßen Umwälzvorrichtung, wie eine Pumpe, Rohre, Leitungen, u.ä., außerhalb des Reaktorraums angeordnet sein können, welche mit anderen, in dem Reaktorraum angeordneten Komponenten, Elemente und/oder Bauteile der erfindungsgemäßen Umwälzvorrichtung zumindest funktionell zusammenwirken und so die Umwälzvorrichtung bilden.

Als die Biomasse bzw. den Biofilm erzeugende Mikroorganismen können insbesondere Sulfat-reduzierende Bakterien wie solche der Spezies der Genera: Desulforomonas sp. (mesophil), Desulfotomaculum KT7 (thermophil), die Spezies Desulforolobus ambivalens, Acidianus infernus, Acidianus brierley, Stygiolobus azoricus (mesophil), Thermoproteus neutrophilus, Thermoproteus tenax, Thermodiscus maritimus (thermophil), Pyrobaculum islandicum, Pyrodictium occultum, Pyrodictium brockii (hyperthermophil) und andere Spezies der Genera Desulfuvibrio, Desulfotomaculum, Desulfomonas, Thermodesulfobacterium, Desulfobulbus, Desulfobacter, Desulfococcus, Desulfonema, Desulfosarcina, Desulfobacterium und Desulforomas (mesophil) verwendet werden.

Auch können - als die Biomasse bzw. den Biofilm erzeugende Mikroorganismen - insbesondere Schwermetall bzw. Metallreduzierende Bakterien wie solche der Spezies der Genera Geobacter, Pseudomonas, Shewanella, Desulfovibrio, Desulfobacterium, Desulfomicrobium, Desulforomonas, Alteromonas verwendet werden.

Mikroorganismen können dabei - zur Erzeugung des Biofilms auf dem Trägermaterial - auf das Trägermaterial angeimpft werden (mikrobielle Startkultur). Auch kann der Biofilm dadurch gebildet werden, dass bereits im Abwasser enthaltenen Mikroorganismen auf das Trägermaterial aufgefiltert werden.

Als das Trägermaterial bzw. Aufwuchskörper eignet sich chemisch und physikalisch inertes, biologisch nicht abbaubares Trägermaterial, beispielsweise ein natürliches Trägermaterial, wie Sand oder (Kiesel-)Steine, oder auch Kunststoffträgermaterial.

Bevorzugt erfolgt die Umwälzung periodisch, beispielsweise jeweils für eine vorgebbare Zeitspanne zu vorgebbaren Zeitpunkten, wie minuten- bzw. stundenweise jeden Tag oder einmal bzw. mehrmals minuten- bzw. stundenweise wöchentlich bzw. monatlich. Entsprechende Kontroll-/Steuerungs-/Regeleinrichtungen für diese periodische Umwälzung können bei der Umwälzvorrichtung vorgesehen sein.

Weiterhin ist bei der erfindungsgemäßen Vorrichtung eine in dem Reaktorraum angeordnete, von dem Umwälzstrom durchströmbare Abscheidvorrichtung vorgesehen, welche eine Filtervorrichtung aufweist, welche an das Trägermaterial angepasste Öffnungen besitzt, wodurch aus dem Umwälzstrom die während der Umwälzung des Umwälzstroms von dem Trägermaterial gelöste Biomasse - und während der Umwälzung des Umwälzstroms ausgeschwemmte, abgelagerte Metallsulfide - aus dem Umwälzstrom ausscheidbar sind.

Die Ablösung der - auf dem Trägermaterial immobilisierten - Biomasse vom Trägermaterial kann dabei durch die bei der Umwälzung des Trägermaterials initiierte Bewegung des Trägermaterials bewirkt werden, beispielsweise auch durch Turbolenzen im Umwälzstrom. Reiben sich mit der Biomasse versetzte Partikel des Trägermaterials bei der Umwälzung gegen- bzw. aneinander, kann sich dadurch die immobilisierte Biomasse - zumindest teilweise - vom Trägermaterial lösen.

An das Trägermaterial angepasste Öffnungen - bei der Filtervorrichtung - kann dabei meinen, dass eine Größe der Öffnungen in der Filtervorrichtung in Abhängigkeit einer Art und/oder Größe des Trägermaterials, wie beispielsweise eine Korngröße oder Kieselsteingröße - bei Sand bzw. Kieselsteinen als Trägermaterial - ausgebildet ist.

Wird so beispeisweise Sand - mit einer bestimmten Korngröße, beispielsweise von ca. 1,1 mm - 1,2 mm - als Trägermaterial verwendet, so können die Öffnungen in der Filtervorrichtung derart bemessen sein, dass sie geringer als die Sandkorngröße sind, beispielsweise ca. 1,0 mm, wodurch das Trägermaterial bzw. der Sand vor einem Durchtritt durch die Filtervorrichtung gehindert bzw. des Trägermaterial bzw. der Sand zurückgehalten wird.

Da aber die - vom Trägermaterial - abgelöste Biomasse - in der Regel - eine geringere Größe/Dimension als das Trägermaterial selbst aufweist, kann diese - im Gegensatz zum Trägermaterial - durch die Öffnungen der Filtervorrichtung durchtreten. D.h., die gelöste Biomasse wird nicht wie das Trägermaterial durch bzw. in der Filtervorrichtung zurückgehalten, wodurch das Trägermaterial bzw. der Sand und die Biomasse (voneinander) getrennt werden.

Kurz, das Trägermaterial einerseits und die Biomasse andererseits werden mittels der Abscheidvorrichtung bzw. der Filtervorrichtung voneinander abgeschieden bzw. die auf dem Trägermaterial immobilisierte und dadurch mit dem Trägermaterial umgewälzte Biomasse wird mittels der Umwälzvorrichtung und der Abscheidvorrichtung von dem Trägermaterial teilweise gelöst (Abrieb) und getrennt.

Die abgeschiedene Biomasse kann dann - aus dem Reaktorraum - abgeführt - und deponiert oder anderweitig (weiter-)verwendet werden, beispielsweise auch wiederverwendet werden durch eine - zumindest teilweise - Rückführung in den Reaktorraum zur Wiederansiedlung/Immobilisierung auf dem Trägermaterial. Hierdurch kann eine schnelle Bildung des Biofilms unterstützt bzw. erreicht werden. Auch kann hierdurch zur gezielten Steuerung des Biofilms/-wachstums und dessen Dicke bzw. der Biofilmerzeugung beigetragen werden.

Die Erfindung erreicht damit eine effiziente, einfache und kostengünstig zu realisierende Abscheidung/Trennung von - immobilisierter - Biomasse - vom Trägermaterial, wodurch sehr effektiv und zielgerichtet Einfluss auf den Biofilm im Reaktorraum genommen werden kann. D.h., mittels der Erfindung kann sehr effizient und in einfacher und kostengünstiger Weise der Biofilm kontrolliert, insbesondere ein (An-)Wachsen des Biofilms und/oder eine Dicke des Biofilms und/oder eine Erneuerung des Biofilm überwacht, - und dem (unerwünschten) Anwachsen bzw. gegebenenfalls einer übermäßigen Dicke des Biofilms entgegengewirkt werden.

Dabei kann die Dicke des Biofilms direkt, wie auch indirekt beschrieben bzw. angegeben und/oder gemessen werden.

So kann unter der Dicke des Biofilms die durchschnittliche an einem Partikel des Trägermaterials angewachsene/immobilisierte Biomasse des Biofilms verstanden werden. Auch die gesamte Menge an Biomasse des Biofilms im Biobett/Umwälzbett im Reaktorraum kann die Dicke des Biofilms beschreiben. Auch kann über eine Proteinkonzentration bzw. Proteinmenge/-masse im Biofilm oder im Abwasser im Reaktorraum auf die Dicke des Biofilms geschlossen werden.

Auch entsprechende Verhältnisgrößen, wie das Verhältnis der gesamten Menge an Biomasse des Biofilms im Biobett zum gesamten Trägermaterial des Biobetts bzw. zur Gesamtmenge aus der gesamten Menge an Biomasse des Biofilms im Biobett und des gesamten Trägermaterials des Biobetts, können die Dicke des Biofilms definieren.

Die Erfindung geht hierbei von der Überlegung aus, dass durch eine zielgerichtete/kontrollierte Umwälzung der auf dem Trägermaterial immobilisierten Biomasse, diese - mittels der Erfindung bei der Umwälzung und Filterung - vom Trägermaterial gelöst und abgeschieden wird, wodurch - mittels der erfindungsgemäßen Umwälzung und Filterung bzw. der Umwälzvorrichtung und der Abscheid- bzw. Filtervorrichtung - gezielt Einfluss auf den Biofilm, dessen Immobilisierung bzw. dessen Anwachsen und/oder dessen Dicke genommen werden kann.

Einfach bzw. anschaulich ausgedrückt, bei jedem Umwälzen des Trägematerials mittels der erfindungsgemäßen Vorrichtung wird die darauf immobilisierte Biomasse vom Trägermaterial - zumindest teilweise - gelöst und abgeschieden, wodurch sich der immobilisierte Biofilm - gezielt und auf einfache Weise - reduzieren lässt.

Damit verhindert die Erfindung eine mit der Zeit zu stark zunehmende Biofilmdicke, welche zu einer Unterversorgung der untersten Biofilmschichten und damit zu deren Absterben führt. Dessen Folge, nämlich ein Ablösen von Teilen des Biofilms bzw. Biomasse komplett vom Trägermaterial, was periodisch zu einem starken Rückgang der Reinigungsleistung führt, kann dadurch die Erfindung verhindern. Die Reinigungsleistung kann so durch die Erfindung verbessert werden.

Auch der Ausbildung von den unerwünschten bevorzugten Fließwegen wirkt die Erfindung entgegen, was auch zu einer Verbesserung der Reinigungsleistung beiträgt.

Im Weiteren vermeidet die Erfindung auch die sonst notwendige regelmäßige Reinigung bzw. Rückspülung und damit verbundene Unterbrechungen des eigentlichen Reinigungsprozesses bei dem Abwasser.

Das erfindungsgemäße Verfahren setzt auf einer Vorrichtung zur Behandlung von Abwässern mit einem Reaktorraum, einer im Wesentlichen in dem Reaktorraum angeordneten Umwälzvorrichtung zu einer, insbesondere periodischen, Umwälzung eines Umwälzstroms aus einem sich in dem Reaktorraum befindenden Abwasser sowie einem in dem Reaktorraum angeordneten Trägermaterial, auf welchem Biomasse eines Biofilms immobilisiert ist, und einer in dem Reaktorraum angeordneten, von dem Umwälzstrom durchströmbaren Abscheidvorrichtung auf.

Im Wesentlichen in dem Reaktorraum angeordnet meint auch hier, dass auch Komponenten, Elemente und/oder Bauteile der erfindungsgemäßen Umwälzvorrichtung, wie eine Pumpe, Rohre, Leitungen, u.ä., außerhalb des Reaktorraums angeordnet sein können, welche mit anderen, in dem Reaktorraum angeordneten Komponenten, Elemente und/oder Bauteile der erfindungsgemäßen Umwälzvorrichtung zumindest funktionell zusammenwirken und so die Umwälzvorrichtung bilden.

Insbesondere kann das erfindungsgemäße Verfahren auf einer erfindungsgemäßen Vorrichtung sowie deren Weiterbildungen aufsetzten.

Bei dem erfindungsgemäßen Verfahren wird der Umwälzstrom unter Verwendung der Umwälzvorrichtung, insbesondere periodisch, in dem Reaktorraum umgewälzt.

Bei der, insbesondere periodischen, Umwälzung des Umwälzstroms wird die auf dem Trägermaterial immobilisierte Biomasse von dem Trägermaterial gelöst. Unter Verwendung der Abscheidvorrichtung wird die gelöste Biomasse aus dem Abwasser abgeschieden.

Erfindungsgemäß ist dabei vorgesehen, dass mittels der Umwälzung der Biofilm, insbesondere ein Anwachsen der auf dem Trägermaterial immobilisierten Biomasse des Biofilms und/oder eine Dicke des Biofilms, kontrolliert wird.

Unter dieser erfindungsgemäßen Kontrolle des Biofilms - über die - zielgerichtete/kontrollierte Umwälzung - versteht die Erfindung, dass über eine gezielte Durchführung - bei dementsprechend vorgegebenen Umwälzparametern, wie Dauer, Häufigkeit, Umwälzflussmenge, Umwälzgeschwindigkeit, Pumprate, Pumpfrequenz, u.ä., - der Umwälzung des Umwälzstroms bzw. der mit dem Trägermaterial immobilisierten Biomasse ein bestimmtes Ergebnis/Ziel bei dem Biofilm im Reaktorraum erreicht werden soll, beispielsweise die Verhinderung des (Weiter-)Anwachsens des Biofilms im Reaktorraum und/oder eine Reduzierung der Dicke des Biofilms. Auch die Erneuerung des Biofilms im Reaktorraum wie auch die Wanderungsgeschwindigkeit des Umwälzbetts, d.h. des Biobetts aus Trägermaterial und immobilisierter Biomasse, im Reaktorraum kann so gezielt durch die Erfindung kontrolliert bzw. reguliert werden.

Die Erfindung geht hierbei von der Überlegung aus, dass durch - bei entsprechend vorgebbaren Umwälzparametern - gezielter Umwälzung der auf dem Trägermaterial immobilisierten Biomasse diese - mittels der Erfindung bei der Umwälzung und Abscheidung - vom Trägermaterial gelöst und abgeschieden wird, wodurch - mittels der Umwälzung und Abscheidung bzw. der Umwälzvorrichtung und der Abscheidvorrichtung gezielt Einfluss auf den Biofilm, dessen Immobilisierung bzw. dessen Anwachsen und/oder dessen Dicke genommen werden kann.

Einfach bzw. anschaulich ausgedrückt, bei jedem "kontrollierten" Umwälzen des Trägematerials bei dem erfindungsgemäßen Verfahren wird die auf dem Trägermaterial immobilisierte Biomasse vom Trägermaterial - zumindest teilweise bzw. partiell - gelöst und abgeschieden, wodurch sich der immobilisierte Biofilm - gezielt/kontrolliert - reduzieren lässt.

Damit verhindert die Erfindung eine mit der Zeit zu stark zunehmende Biofilmdicke, welche zu einer Unterversorgung der untersten Biofilmschichten und damit zu deren Absterben führt. Dessen Folge, nämlich ein Ablösen von Teilen des Biofilms bzw. Biomasse komplett vom Trägermaterial, was periodisch zu einem starken Rückgang der Reinigungsleistung führt, kann die Erfindung dadurch verhindern. Die Reinigungsleistung kann so durch die Erfindung verbessert werden.

Im Weiteren vermeidet die Erfindung auch die sonst notwendige regelmäßige Reinigung bzw. Rückspülung und damit verbundene Unterbrechungen des eigentlichen Reinigungsprozesses bei dem Abwasser.

Darüber hinaus verhindert die Erfindung - als weiteren Effekt der erfindungsgemäßen Umwälzung und Abscheidung - die - bei der Abwasserbehandlung im Reaktorraum aufgrund der Ausfällung und Ablagerungen von Metallsulfiden auftretenden - Verblockung von den offenen Poren im Biobett - und damit auch die Ausbildung bevorzugter Fließwege. Auch werden solche Sulfide, die sich auf dem Trägermaterial und dem Biofilm abgesetzt haben und somit den Stoffaustausch zwischen dem zu reinigendem Abwasser und dem Biofilm hemmen, durch die erfindungsgemäße Umwälzung und Abscheidung beseitigt.

Weiter mitumfasst bei der Erfindung sei ein Verfahren zur Behandlung von Abwässern, insbesondere von sulfat- und/oder schwermetallhaltigen Minenwässern, bei dem bei einem Abwasser, insbesondere bei einem sulfat- und/oder schwermetallhaltigen Minenwasser, zunächst ein Membranverfahren, insbesondere eine Nanofiltration, und anschließend bei einem bei dem Membranverfahren, insbesondere bei der Nanofiltration, erzeugten Konzentrat eine biologische Sulfatreduktion durchgeführt wird.

Kurz, die Erfindung koppelt hier, beispielsweise für die Reinigung sulfat- und metallhaltiger Bergbau- und anderer Abwässer, eine Membranfiltration, insbesondere eine Nanofiltration, (vorgeschaltet) mit einer - im Allgemeinen - biologischen Sulfatreduktion (nachgeschaltet).

Diese Kombination von - vorgeschalteter - Membranfiltration bzw. Nanofiltration und - nachfolgend am Konzentrat durchgeführter - allgemeiner biologischen Sulfatreduktion erreicht eine Verringerung der - bei der nachgeschalteten biologischen Abwasserbehandlung - zu behandelnden Abwassermenge. Hier für die biologische Sulfatreduktion einsetzbare Reaktoren können dadurch kompakt gebaut sein, was Bau- und Investitionskosten spart.

Auch können hierdurch die Kosten der Membran- bzw. Nanofiltrationsstufe begrenzt werden, da dort immer eine gewisse Restmenge an Konzentrat übrigbleibt, das wirtschaftlich nicht weiter durch diese Filtrationstechnik aufgearbeitet werden kann. Eine kostenintensive Deponierung oder Weiterbehandlung durch andere Technologien wäre notwendig.

Eine - ökologisch und ökonomisch sinnvolle - Aufbereitung wird so dann durch die nachgeschaltete biologische Sulfatreduktion erreicht, wobei es insbesondere zu einer weiteren Reduktion der Sulfat- und Metallkonzentrationen kommt.

Hier kann weiter besonders zweckmäßig vorgesehen sein, dass die - nachgeschaltete - biologische Sulfatreduktion unter Verwendung der erfindungsgemäßen Vorrichtung - und einer deren Weiterbildungen - und/oder nach dem erfindungsgemäßen Verfahren - und eines dessen Weiterbildungen - durchgeführt wird.

Nach einer bevorzugten Weiterbildung weist die Filtervorrichtung ein die Öffnungen aufweisendes Gitterelement oder ein die Öffnungen aufweisendes Lochblech auf. Die Öffnungen bzw. die Löcher können derart bemessen sein, dass das Trägermaterial vor einem Durchtritt durch das Gitterelement bzw. Lochblech zurückgehalten wird. Vereinfacht ausgedrückt, die Öffnungen/Löcher im Gitterelement bzw. Lochblech sind kleiner als die Partikelgröße des Trägermaterials.

Weiter kann vorgesehen sein, dass die Abscheidvorrichtung einen von dem Umwälzstrom in einer Durchströmungsrichtung durchströmbaren Schlammabsetztrichter mit einem Trichterhals und einem Trichtertopf aufweist. An dem Trichtertopf kann die Filtervorrichtung angeordnet sein. Auch ist es zweckmäßig, dass an dem Schlammabsetztrichter ein Schlammabzug vorgesehen ist, über welchen ausgefiltertes Material, d.h. die - nicht von der Filtervorrichtung zurückgehaltene - Biomasse, wie aber auch beispielsweise ausgefilterte, d.h. nicht von der Filtervorrichtung zurückgehaltene, Fällungsprodukte, abgezogen werden können.

Auch kann vorgesehen sein, dass die Umwälzvorrichtung ein von dem Umwälzstrom in der Durchströmungsrichtung durchströmbares Steigrohr aufweist. An dessen - in der Durchströmungsrichtung - stromabwärtigen Ende kann ein an seiner Außenseite profilierter Rohrausatz aufgesteckt sein.

Nach einer weiteren bevorzugten Weiterbildung umfasst der Trichterhals das - in der Durchströmungsrichtung - stromabwärtige Ende des Steigrohres koaxial.

Dabei meint "koaxial umfasst" auch, dass das - "innenliegendes" Steigrohrende und der - "außenliegende" - das Steigrohr umfassende Trichterhals - im Bereich ihrer Umfassung - voneinander beabstandet sein können. Durch den sich dadurch ergebenden Zwischenraum - zwischen Steigrohrende und Trichterhals - kann Flüssigkeit, wie das Abwasser, und/oder Partikel, wie das - von der Abscheidvorrichtung zurückgehaltene - Trägermaterial, durchtreten.

Bei einer weiteren bevorzugten Weiterbildung ist vorgesehen, dass das Steigrohr einen auf das Steigrohr aufgesteckten Trichter aufweist, wobei dieser Trichter derart auf das Steigrohr aufgesteckt ist, dass eine Öffnung eines Trichtertopfes des Trichters entgegen der Durchströmungsrichtung des Steigrohrs geöffnet ist.

Weiter kann auch vorgesehen sein, dass die Umwälzvorrichtung eine Vorrichtung nach einem Prinzip einer Mammutpumpe aufweist. Anschaulich bzw. vereinfacht ausgedrückt, die Umwälzvorrichtung, welche dem Umwälzstrom umwälzt, kann mittels einer Mammutpumpe betrieben werden.

Diese Vorrichtung nach dem Prinzip einer Mammutpumpe bzw. die Mammutpumpe kann - weiter auch - mittels einer Membranpumpe betrieben werden.

Eine solche Mammutpumpe und Membranpumpe sind im Stand der Technik bekannt und einfach und kostengünstig zu realisieren.

Hier kann auch vorgesehen sein, dass die Membranpumpe mit einem Gas, insbesondere Stickstoff oder Kohlendioxid, oder einer Flüssigkeit, insbesondere dem Abwasser oder bereits einem mittels/durch der Erfindung gereinigten Abwasser betrieben wird.

Das Gas oder die Flüssigkeit kann dabei in einem Bereich am - in der Durchströmungsrichtung - stromaufwärtigen Ende des Steigrohres in das Steigrohr eingepresst werden.

Weiterhin kann auch vorgesehen sein, dass über eine Pumprate und/oder Pumpfrequenz der Vorrichtung nach dem Prinzip der Mammutpumpe und/oder der Membranpumpe die Umwälzung des Trägermaterials kontrollierbar ist. Vereinfacht ausgedrückt, über die Pumprate und/oder die Pumpfrequenz wird eine Wanderungsgeschwindigkeit des Biobetts/Umwälzbetts bzw. die Umwälzung des Umwälzbetts reguliert. Eine Biofilmerneuerung kann so auch über die Wanderungsgeschwindigkeit des Biobetts reguliert werden.

Auch können hier Markierungen am Trägermaterial vorgesehen sein, welche einen Umwälzstatus bei dem Umwälzbett/Biobett kenntlich machen. Beispielsweise kann eine Schicht des Trägermaterials (farblich) markiert sein, wodurch sich die Bewegung/Umwälzung des Biobetts beobachten und kontrollieren lässt.

Nach einer weiteren bevorzugten Weiterbildung weist der Reaktorraum einen - an bzw. in diesem - angeordneten Zufluss für einen Zufluss des - zu behandelnden - Abwassers in den Reaktorraum auf.

Auch kann der Reaktorraum einen an bzw. in diesem angeordneten Ablauf für einen Ablauf von in der Vorrichtung bzw. im Reaktorraum behandeltem Abwasser aus dem Reaktorraum aufweisen. Bevorzugt sieht der Ablauf einen Überströmrand vor, über welchen das behandelte Abwasser aus dem Reaktorraum abziehbar ist.

Eine Größe bzw. ein Volumen des - meist als ein im Wesentlichen hohlzylindrischer Körper gestalteten - Reaktorraums kann in weiten Bereichen beliebig sein. Insbesondere kann das Volumen des Reaktorraums in Abhängigkeit der - wie gewünscht - zu behandelnden Abwassermenge bemessen sein. Beispeisweise eignen sich Reaktorräume mit Volumina von wenigen Litern bis zu vielen Kubikmetern, insbesondere mit Volumina im Bereich von 500 l - 3000 l, ganz besonders im Bereich von 1500 l-2500 l.

Weiterhin kann vorgesehen sein, die Erfindung bei einem Biobettreaktor mit periodisch umgewälztem Biobett zu verwenden bzw. einzusetzen. Das Biobett - im Reaktorraum - wird dabei durch das Trägermaterial sowie durch die auf dem Trägermaterial immobilisierte Biomasse des Biofilms gebildet, welches mittels der Umwälzvorrichtung periodisch umgewälzt wird.

Die periodische Umwälzung kann hier jeweils für eine vorgebbare Zeitspanne zu vorgebbaren Zeitpunkten, wie minuten- bzw. stundenweise jeden Tag oder einmal bzw. mehrmals minuten- bzw. stundenweise wöchentlich bzw. monatlich erfolgen.

Entsprechende Kontroll-/Steuerungs-/Regeleinrichtungen für diese periodische Umwälzung können bei der Umwälzvorrichtung vorgesehen sein.

Auch kann - insbesondere über angepasste Regulierung des Zuflusses des Abwassers, des Abflusses des behandelten Abwassers, über Regulierung der Umwälzung bzw. deren Parameter, der (konstruktiven) Gestaltung des Reaktorraums und/oder Dimensionierung der Abscheidvorrichtung und anderer Durchflusselemente - eine Verweildauer des Abwassers im Biobett bzw. im Porenraum des Biobetts von 30 min - 5 h, insbesondere von 1 h - 3 h, im Weiteren im Besonderen ca. 2 h, vorgesehen werden. Verweildauern können nach bedarf auch durchaus länger sein.

Nach einer weiteren bevorzugten Weiterbildung ist vorgesehen, dass das Trägermaterial zu einem Start der Vorrichtung durch ein Animpfen mit Mikroorganismen besiedelt wird (mikrobielle Startkultur). Auch kann vorgesehen sein, dass die mikrobielle Startkultur durch ein Auffiltern von bereits im Abwasser enthaltenen Mikroorganismen auf das Trägermaterial hergestellt wird.

Nach einer anderen bevorzugten Weiterbildung ist vorgesehen, dass ein Redoxpotenzial des Abwassers verringert wird. Diese Verringerung kann beispielsweise durch Zugabe von reduzierten Schwefelverbindungen, wie Sulfide (Natriumdisulfid), zu dem Abwasser bewirkt werden. Ein reduziertes Redoxpotenzial des Abwassers verstärkt dabei die biologische Aktivität und/oder erleichtert den Start des Verfahrens.

Eine entsprechende - hier auch einsetzbare - Sensorik zur Messung des Redoxpotenzials einer Flüssigkeit stellt der Stand der Technik zur Verfügung.

Auch kann vorgesehen sein, dass dem Abwasser Nährstoffe, insbesondere eine Kohlenstoff-Quelle, zugegeben werden, welche an die Mikroorganismen, welche den Biofilm erzeugen, angepasst sind. Insbesondere eignet sich hier - als eine solche Kohlenstoff-Quelle (Elektronendonor) - Methanol, Ethanol oder Lactat.

Bei einer anderen bevorzugten Weiterbildung wird bei dem behandelten Abwasser eine Nachbehandlung, beispielsweise eine biologische Oxidation zu Schwefel oder ein Ausstrippen von Schwefelwasserstoff, durchgeführt. Der ausgestrippte Schwefelwasserstoff kann dabei als Fällungschemikalie weiterverwendet werden.

Auch kann nach einer besonders bevorzugten Weiterbildung vorgesehen werden, dass bei dem Abwasser - vor der Behandlung des Abwassers in dem Reaktorraum und außerhalb des Reaktorraums - eine biologische Oxidation durchgeführt wird (vorgeschaltete biologische Oxidationsstufe).

Bei der biologischen Oxidation können in dem Abwasser enthaltene Metalle, insbesondere Eisen, oxidiert und anschließend das oxidierte Metall aus dem Abwasser abgetrennt werden. Im Speziellen kann zweiwertiges Eisen zu Eisen(III) oxidiert und dessen Ausfällung z.B. als Schwermannit aus dem Abwasser abgetrennt werden.

Dadurch wird die Eisenkonzentration im Abwasser vor dessen Eintritt in den Reaktorraum deutlich gesenkt, wodurch es dort zu wesentlich weniger Verblockungen des Biofilms auf dem Trägermaterial und weniger Verblockungen von Fließwegen durch das Bett des Trägermaterials kommt. Dieses führt wiederum zu einem deutlich höheren Stoffaustausch zwischen dem Biofilm und dem zu behandelndem Abwasser und damit zu einer verbesserten Reinigungsleistung im Reaktorraum.

Ferner kann es hier auch zweckmäßig sein, das in der vorgeschalteten biologischen Oxidationsstufe abgetrennte, oxidierte Metall einer rohstofflichen Nutzung oder einer Deponierung zuzuführen.

Die vorgeschaltete Metallabtrennung kann - neben dieser biologischen Metallabtrennung - auch rein chemisch, z.B. durch Nutzung von im Bioreaktor gebildetem Schwefelwasserstoff bzw. allein durch Anhebung des ph-Wertes des Abwassers, erfolgen.

Weiterhin kann bei der vorgeschalteten Metallabtrennung auch vorgesehen werden, diese bei einem vorgegebenen pH-Wert, beispielsweise von ungefähr 3, im Abwasser durchzuführen. Hier können dann in einem einzigen Verfahrensschritt - überwiegend ein ganz bestimmtes Metall, aber auch weitestgehend viele bzw. alle Metalle ausgefällt bzw. abgetrennt werden.

Es kann aber auch vorgesehen werden, die vorgeschaltete Metallabtrennung jeweils bei unterschiedlich eingestellten pH-Werten des Abwassers durchzuführen, wodurch bei einem jeweils eingestellten pH-Wert im Abwasser gezielt ein bestimmtes Metall im Abwasser ausgefällt wird. Hierdurch können schrittweise einzelne Metalle abgetrennt werden.

Bei einer besonders bevorzugten Weiterbildung ist vorgesehen, dass bei dem Abwasser vor der Behandlung des Abwassers in dem Reaktorraum eine Membranfiltration, insbesondere eine Nanofiltration, durchgeführt wird. Ein durch die Membranfiltration, insbesondere die Nanofiltration, erzeugtes Konzentrat wird dann als das Abwasser dem Reaktorraum zur dortigen biologischen Behandlung zugeführt.

Durch diese Kombination von Membranfiltration bzw. Nanofiltration mit der biologischen Abwasserbehandlung wird eine Verringerung der - bei der biologischen Abwasserbehandlung - zu behandelnden Abwassermenge erreicht. Der Reaktorraum kann dadurch kompakt gebaut werden, was Bau- und Investitionskosten spart.

Somit können hier auch die Kosten der Membran- bzw. Nanofiltrationsstufe begrenzt werden, da dort immer eine gewisse Restmenge an Konzentrat übrigbleibt, das wirtschaftlich nicht weiter durch diese Filtrationstechnik aufgearbeitet werden kann. Eine kostenintensive Deponierung oder Weiterbehandlung durch andere Technologien wäre notwendig.

Eine - ökologisch und ökonomisch sinnvolle - Aufbereitung wird so dann durch die nachgeschaltete erfindungsgemäße biologische Behandlung erreicht, wobei es insbesondere - im Falle von sulfat- und metallhaltigen Abwässern - zu einer weiteren Reduktion der Sulfat- und Metallkonzentrationen kommt.

Der Prozess der (mikro-)biologischen Abwasserbehandlung ist ein Vorgang, der unter bestimmten chemischen Umwelt-/Randbedingungen bzw. -parameter von dazu befähigten Mikroorganismen durchgeführt wird. Weichen bestimmte, für diesen Prozess notwendige Bedingungen bzw. Parameter - in einem gewissen Maß - von den für die Mikroorganismen optimalen Bedingungen ab, kann es zu einer Beeinträchtigung des biologischen Reinigungsprozesses bis hin zu einem vollständigen Versagen des biologischen Prozesses kommen.

Ein Beispiel hierfür stellt die Konkurrenz um die zum Wachstum von sulfatreduzierenden Mikroorganismen eingesetzten Nährstoffe bzw. eingesetzten Substrate, wie beispielsweise Methanol, dar.

So können nicht auf sulfatreduzierende Mikroorganismen abgestimmte Parameter dazu führen, dass sich konkurrierende Mikroorganismen in einem Bioreaktor stärker anreichern.

Dies kann aufgrund der Konkurrenz der unterschiedlichen Mikroorganismen zu einer ineffizienten Nutzung der verwendeten Nährstoffe bzw. verwendeten Substrats für die Sulfatreduktion führen.

Dadurch werden wiederum - wegen des erhöhten Nährstoff- bzw. Substratverbrauchs - höhere Kosten für das Verfahren der biologischen Sulfatreduktion verursacht. Längerfristig kann es aufgrund der zunehmenden Dominanz der - konkurrierenden bzw. nicht sulfatreduzierenden - Mikroorganismen zu einem fast vollständigen Erliegen der biologischen Sulfatreduktion kommen.

Aus diesem Grund ist ein rechtzeitiges Erkennen negativer Entwicklungen bei dem Verfahren sowie eine gezielte Kontrolle des Verfahrens - mittels Messung und/oder gezielter Kontrolle/Steuerung/Regelung von Betriebsparametern von großer Wichtigkeit für einen stabilen und effizienten Betrieb.

So ist bei einer weiteren bevorzugten Weiterbildung vorgesehen, dass bei dem im Reaktorraum befindlichen Abwasser und/oder bei dem behandelten Abwasser ein physikalischer und/oder chemischer Parameter, insbesondere ein pH-Wert, eine Temperatur, ein Redoxpotential, eine Sulfatkonzentration, eine Substratkonzentration (Elektronendonatorkonzentration), eine Nährstoffkonzentration, eine Konzentration von durch die Mikroorganismen erzeugten Abbauprodukten und/oder ein Umfang bzw. eine Menge der Mikroorganismen, oder mehrere solche physikalische und/oder chemische Parameter bestimmt, insbesondere kontinuierlich gemessen, wird bzw. werden.

Weiter kann hier vorgesehen sein, dass der oder die mehreren physikalischen und/oder chemischen Parametern an bzw. jeweils an unterschiedlichen Positionen in dem Reaktorraum bestimmt, insbesondere kontinuierlich gemessen, wird bzw. werden.

Unter Verwendung des oder der bestimmten, insbesondere kontinuierlich gemessenen, physikalischen und/oder chemischen Parameter kann die Behandlung des Abwassers in dem Reaktorraum, insbesondere durch Einstellung der Umwälzung, gesteuert werden.

Nach einer weiteren bevorzugten Weiterbildung kann vorgesehen sein, dass bei dem im Reaktorraum befindlichen Abwasser und/oder bei dem behandelten Abwasser ein Umfang bzw. eine Menge von mit den Mikroorganismen konkurrierenden (Mikro-)Organismen und/oder von Abbauprodukten von den mit den Mikroorganismen konkurrierenden (Mikro-)Organismen, insbesondere eine Konzentration von organischen Carbonsäuren (Essigsäure, Buttersäure) (fermentierende Mikroorganismen) oder eine Methankonzentration (methanogene Mikroorganismen), bestimmt, insbesondere kontinuierlich gemessen, wird.

Unter Verwendung der bestimmten Konzentration der von den Mikroorganismen erzeugten Abbauprodukte und/oder der bestimmten Konzentration der von den mit den Mikroorganismen konkurrierenden (Mikro-)Organismen erzeugten Abbauprodukte kann eine Behandlungsumgebung des Abwassers im Reaktorraum, insbesondere Biofilm, kontrolliert werden.

Auch kann ein Verhältnis zwischen den Biofilm erzeugenden Mikroorganismen und den mit den Biofilm erzeugenden Mikroorganismen konkurrierenden (Mikro-)Organismen überwacht werden, insbesondere gesteuert und/oder geregelt werden. Dies kann insbesondere dadurch erfolgen, dass eine Aufenthaltsdauer des Abwassers im Reaktorraum bzw. im Biobett geregelt wird, der pH-Wert des Abwassers im Reaktorraum geregelt/reguliert wird, Sauerstoff, insbesondere kurzzeitig, im Reaktorraum dosiert wird und/oder das Verhältnis der Substratkonzentration zur Sulfatkonzentration im Abwasser gesteuert bzw. reguliert wird.

Ferner ist nach einer besonders bevorzugten Weiterbildung vorgesehen, dass die Erfindung zu einer Behandlung, insbesondere Reinigung, des Abwassers eingesetzt wird, wobei das Abwasser mit dem auf dem Trägermaterial immobilisierten Biofilm in Kontakt gebracht wird und dabei Fällungsprodukte, insbesondere Metallsulfide, aus dem Abwasser ausgefällt und die Fällungsprodukte, insbesondere die ausgefällten Metallsulfide, bei der Umwälzung - insbesondere mittels einer Sedimentation- und/oder der Filterungsvorrichtung - abgeschieden werden, wodurch das Abwasser behandelt bzw. gereinigt wird. Auch kann durch die Erfindung der Biofilm, insbesondere periodisch, erneuert werden, wobei die Erneuerung des Biofilms über die Umwälzung reguliert wird, insbesondere durch eine über die Umwälzung regulierte Biobettwanderungsgeschwindigkeit.

Mittels der erfindungsgemäßen Vorrichtung bzw. durch das erfindungsgemäße Verfahren können insbesondere Minenwässer aus Bergbaubetrieben aufgearbeitet bzw. gereinigt werden.

So ist die Erfindung insbesondere geeignet zur Aufarbeitung von "saurem Minenablauf" (acid mine drainage AMD) oder "saurem Gesteinsablauf" (acid rock drainage ARD) oder zur Aufarbeitung von mit anderen Verfahren, wie beispielsweise einer Membranfiltration, aufkonzentrierten Minenwässern. D.h., die Erfindung kann nicht nur zur direkten Behandlung von Minenwässern eingesetzt werden, sondern auch Einsatz bei der Behandlung von Minenwasserkonzentraten, beispielsweise aus einer Direktfiltration, Verwendung finden, insbesondere da durch eine Vorfiltration eine erhebliche Reduktion des zu behandelnden Volumenstroms erfolgt. Außerdem bringt die Erhöhung der Metallgehalte eine erhöhte Wirtschaftlichkeit im Falle der vorgeschalteten Fällung mit sich.

Auch kann das erfindungsgemäße Verfahren in der metallverarbeitenden Industrie bei der Aufbereitung, insbesondere von dort auftretenden metallhaltigen (Industrie-)Abwassern, eingesetzt werden.

Die bisher gegebene Beschreibung vorteilhafter Ausgestaltungen der Erfindung enthält zahlreiche Merkmale, die in den einzelnen Unteransprüchen teilweise zu mehreren zusammengefasst wiedergegeben sind. Diese Merkmale wird der Fachmann jedoch zweckmäßigerweise auch einzeln betrachten und zu sinnvollen weiteren Kombinationen zusammenfassen.

In Figuren sind Ausführungsbeispiele der Erfindung dargestellt, welche im Weiteren näher erläutert werden. Gleiche Bezugszeichen in den Figuren bezeichnen technisch gleiche Elemente. Pfeile verdeutlichen Bewegungsrichtungen von Objekten bzw. Elementen.

Es zeigen:
- FIG 1: einen Bioreaktor mit periodisch umgewälztem Sandbett (Umwälzbett) gemäß einem Ausführungsbeispiel einer Vorrichtung zur Behandlung von Abwässern, insbesondere von sulfat- und/oder schwermetallhaltigen Minenwässern,
- FIG 2: ein Steigrohr für den Bioreaktor mit periodisch umgewälztem Sandbett (Umwälzbett) gemäß dem Ausführungsbeispiel einer Vorrichtung zur Behandlung von Abwässern, insbesondere von sulfat- und/oder schwermetallhaltigen Minenwässern,
- FIG 3: einen Schlammtrichter für den Bioreaktor mit periodisch umgewälztem Sandbett (Umwälzbett) gemäß dem Ausführungsbeispiel einer Vorrichtung zur Behandlung von Abwässern, insbesondere von sulfat- und/oder schwermetallhaltigen Minenwässern,
- FIG 4: den Bioreaktor mit Verdeutlichung dortiger Prozessvorgänge, insbesondere des periodisch umgewälzten Sandbetts (Umwälzbett),
- FIG 5: einen erfindungsgemäßen Verfahrenablauf mit einer der Behandlung des Minenwassers in dem Bioreaktor vorgeschalteten biologischen Oxidation,
- FIG 6: eine Behandlung von Abwässern, insbesondere von sulfat- und/oder schwermetallhaltigen Minenwässern, mit einer zunächst durchgeführten Membranfiltration, insbesondere einer Nanofiltration, und einer nachfolgend durchgeführten biologischen Sulfatreduktion,
- FIG 7: eine über die Kontrolle der mikrobiologischen Lebensgemeinschaft und deren Prozessbedingungen im Reaktor gesteuerte Behandlung von Abwässern, insbesondere von sulfat- und/oder schwermetallhaltigen Minenwässern.

Abwasserbehandlung von sulfat- und schwermetallhaltigen Minenwässern in einem Bioreaktor mit periodisch umgewälztem, biologischem Sandbett

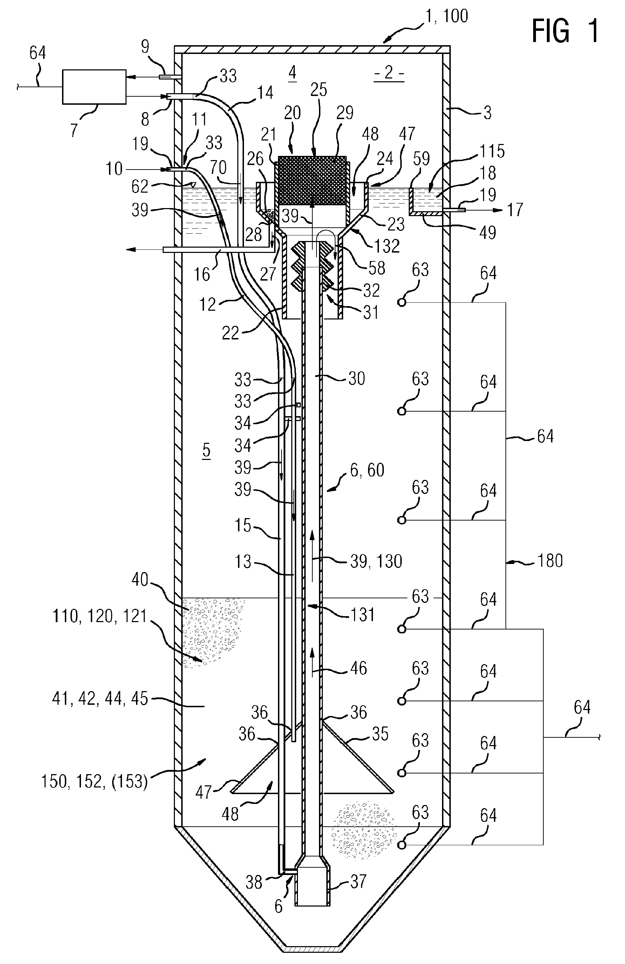

Fig. 1 zeigt einen Bioreaktor 1 (kurz nur Reaktor 1) mit periodisch umgewälztem biologischem Sandbett (Umwälzbett) 40 zur Abwasserbehandlung 100 von sulfat- und schwermetallhaltigen Minenwässern 10 (kurz nur Minenwässer bzw. Minenwasser 10 oder Abwasser bzw. Abwässer 10).

Der Reaktor 1 weist, wie Fig. 1 zeigt, eine im Wesentlichen, durch einen Hohlzylinder gebildete zylindrische Form mit einem konisch zulaufenden unteren Ende auf, welche ein einen Reaktorraum 2 umschließendes, geschlossenes (Reaktor-) Gehäuse 3 des Reaktors 1 - mit einem Fassungsvermögen von ca. 2000 l - bildet.

Im unteren Bereich des Reaktors 1 befindet sich das Umwälzbett 40, d.h. das Sandbett 40 aus Sandpartikel 41 (Trägermaterial 41), in welchem bzw. auf welchen Mikroorganismen 45 eine reduzierende Biomasse 45 eines Biofilms 44 - als Ergebnis einer mikrobiologischen bzw. bakteriellen Biofilmbildung - immobilisiert ist.

Vor dem Start des Reaktors 1 wurde dabei das Trägermaterial durch eine mikrobielle Startkultur besiedelt 152. Alternativ hierzu kann die mikrobielle Startkultur auch durch Auffiltern von bereits im Minenwasser 10 enthaltenen Mikroorganismen 45 hergestellt werden 153.

Um die Biofilmbildung im Reaktor 1 - auch wie hier zum Start des Reaktors 1 - weiter zu beschleunigen, ist die Verringerung des Redoxpotenzials des Minenwassers 10 durch Zugabe von Natriumdisulfid 150 vorgesehen (nicht dargestellt).

In diesem mit dem Biofilm 44 versetzten bzw. mit den Mikroorganismen 45 besiedeltem - und von dem zu behandelnden bzw. zu reinigenden Minenwasser 10 durchströmten 110 - Sandbett 40 erfolgt die (biologische) Reinigung bzw. Behandlung des sulfat- und schwermetallhaltigen Minenwassers 10, 100.

Die Reinigungswirkung beruht dabei auf der biologischen Reduktion von Sulfat bzw. Metallen (im Minenwasser 10) durch die Biomasse 45, 120.

Mittels der Biomasse 45 bzw. der (Sulfat-/Metall-)reduzierende Bakterien 44 erfolgt eine Sulfatreduzierung zu Sulfid und damit eine einhergehende Fällung von Metallsulfiden 42, 120 sowie eine biologische Reduktion der (Schwer-)Metalle zu einem geringeren Ladungszustand (Valenz), in welcher Form das Metall als Metallsulfid, Metallcarbonat, Metalloxid oder Hydroxid, Metallphosphat 42 oder als das elementare Metall 42 ausfällt 120 (kurz allesamt als Ausfällungen oder Fällungsprodukte 42 bezeichnet).

Es werden dem Abwasser 10 organische Substanzen, wie Methanol, Ethanol und/oder Lactat (primäre Kohlenstoffquelle und Elektronendonor), zugegeben, deren Oxidation Elektronen zur Reduktion des Sulfats bereitzustellen.

Die Ausfällungen 42 werden im Sandbett 40 zurückgehalten, wobei diese das Trägermaterial 41 verkrusten 121. Das behandelte/gereinigte Abwasser 17 wird mittels eines Ablaufs 18 aus dem Reaktor 1 abgezogen 115.

Im oberen Bereich des Reaktors 1 weist das Reaktorgehäuse 3 einen - durch einen Rohrstutzen 19 und im Weiteren durch eine Schlauchverbindung 12 und ein Rohr 13 gebildeten - Zufluss 11 auf, über welchen - in Durchströmungsrichtung 39 - das Minenwasser 10 dem Reaktor 1 zugeführt bzw. in den unteren Bereich des Reaktorraum 2 eingeleitet wird.

In etwa derselben Höhe des Zufluss-Rohrstutzens 19 in der Reaktorgehäusewand ist der Abfluss/Ablauf 18 für den Abfluss des im Reaktor 1 behandelten bzw. biologisch gereinigten Abwassers 17 angeordnet. Dieser Abfluss 18 wird, wie Fig. 1 zeigt, gebildet durch eine an der Reaktorgehäusewand angeordnete Ablaufrinne 49 mit in der Gehäusewand angeordnetem Rohrstutzen 19.

Das - zu behandelnde - Minenwasser 10 wird mittels einer Pumpe (nicht dargestellt) unter Druck (in den unteren Bereich) in den Reaktor 1 geleitet und durchströmt das Sandbett 40 von unten nach oben; das behandelte Abwasser 17 wird gegebenenfalls mittels einer weiteren Pumpe (nicht dargestellt) - oder als freier Abfluss - über den Überströmrand 59 der Ablaufrinne 49 und dem Rohrstutzen 19 aus dem Reaktor 1 abgezogen.

Durch Steuerung bzw. Regulierung des Zuflusses/-menge des Abwassers 10 bzw. Abflusses/-menge des behandelten Abwassers 17 wird der Flüssigkeitsspiegel/-pegel in etwa knapp oberhalb des Überströmrandes 59 der Ablaufrinne 49 gehalten.

Oberhalb des Flüssigkeitspegels 62 bildet sich - im Betrieb des Reaktors 1 - der Gasraum 4 im Reaktorraum 2, darunter der Flüssigkeitsraum 5 aus.

Zentral im Reaktorraum 2 ist - als Teil einer einen Umwälzstrom 46 umwälzenden Umwälzvorrichtung 60 - ein Steigrohr 30 angeordnet, welches - bei aktiver, durch die Umwälzvorrichtung 60 betriebener Umwälzung 130 - von dem Umwälzstrom 46 in Durchströmungsrichtung 39 durchströmt wird.

Das Steigrohr 30 ist dabei, wie Fig.1 zeigt, an seinem stromabwärtigen (unteren) Ende offen und mit diesem vom Boden des Reaktors 1 beabstandet angeordnet.

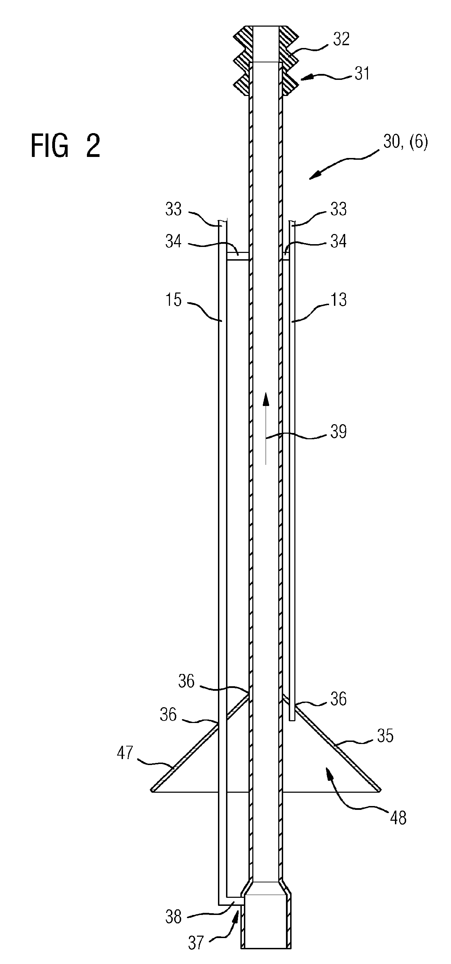

Fig. 2 zeigt - neben Fig. 1 - dieses Steigrohr 30 im Detail.

Das Steigrohr 30 ist - wie Fig.en 1 und 2 zeigen - ein beidseitig offenes Kupferrohr. Andere Rohrmaterialien bei dem Steigrohr 30 sind denkbar.

Am - bezüglich der Durchströmungsrichtung 39 - stromaufwärtigen (unteren) Ende des Steigrohrs 30 weist dieses einen erweiterten Querschnitt auf; am stromabwärtigen (oberen) Ende 31 des Steigrohres 30 ist ein an seiner Außenseite profilierter Rohraufsatz 32 (aus PVC) auf das Steigrohr 30 aufgesteckt. Dabei überragt der Rohraufsatz 32 das Steigrohr 30 um etwa seine halbe Länge.

Ebenfalls im unteren Bereich des Steigrohrs 30 ist ein PVC-Trichter 35 mit einem Trichtertopf 47 mit einer entgegen der Durchströmungsrichtung 39 ausgerichteten Trichter(-topf-)öffnung 48 auf das Steigrohr 30 aufgesteckt.

Neben der Aufstecköffnung/Durchtrittsöffnung 36 für das Steigrohr 30 weist der Trichter 35 - wie Figuren 1 und 2 zeigen - zwei weitere Durchstecköffnungen 36 für zwei der Umwälzvorrichtung 60 bzw. dem Zufluss 11 zugehörige, zu dem Steigrohr 30 parallel ausgerichtete Rohre 13, 15, auf, welche jeweils mittels eines Steges 34 an der Außenseite des Steigrohrs 30 befestigt sind.

Alle Durchsteck-/Durchtrittsöffnungen 36 im Trichter 35 sind dabei abgedichtet.

Das näher an dem Steigrohr liegende (Kupfer-)Rohr 13 ist dem Zufluss 11 zugehörig. An seinem - in Horizontalausrichtung gesehen - oberen Ende ist das dem Zufluss 11 zugehörige Rohr 13 über die Schlauchverbindung 12 - (beiderseits) befestigt über eine Schlauchtülle 33 - mit dem Rohrstutzen 19 des Zufluss 11 verbunden, sodass es von dem - über den Zufluss 11 bzw. den Rohrstutzen 19 - in den Reaktor 1 einströmende Abwasser 10 in der Durchströmungsrichtung 39 (- in Horizontalausrichtung gesehen - von oben nach unten) durchströmt wird.

Das - in Horizontalausrichtung gesehene - untere bzw. - in Durchströmungsrichtung 39 - stromabwärtige Ende des Rohres 13 ragt in die Trichter(-topf-)öffnung 48 und verbleibt dort offen, wodurch das über den Zufluss 11 einströmende Abwasser in die nach unten gerichtete Trichteröffnung eintreten kann. Das - bezüglich des Steigrohres 30 in Bezug auf das Rohr 13 - von dem Steigrohr 30 weiter entfernt angeordnete weitere (Kupfer-)Rohr 15 ist der Umwälzvorrichtung 60 zugehörig. An seinem - in Horizontalausrichtung gesehen - oberen Ende ist das der Umwälzvorrichtung 60 zugehörige Rohr 15 über eine Schlauchverbindung 14 - (beiderseits) befestigt über eine Schlauchtülle 33 - mit einem in der Reaktorgehäusewand angeordneten Zuströmstutzen 8 verbunden.

An seinem - in Horizontalausrichtung gesehenen - unteren Ende ist das der Umwälzvorrichtung 60 zugehörige Rohr 15 über ein Anschlussstück 38 - im Bereich der Querschnittserweiterung 37 des Steigrohres 30 - mit diesem verbunden.

Weiter der Umwälzvorrichtung 60 zugehörig ist - im Bereich des Gasraums 4 des Reaktors 1 - oberhalb des Zuströmstutzens 8 ein weiterer Rohrstutzen 9, d.h. ein Abströmstutzen 9, in der Reaktorgehäusewand angeordnet.

Zuströmstutzen 8 und Abströmstutzen 9 sind jeweils mit einer - mittels Stickstoff 70 betriebenen und außerhalb des Reaktors 1 befindlichen - Membranpumpe 7 verbunden, welche den Stickstoff 70 in Durchströmungsrichtung 39 über den Zuströmungsstutzen 8, dem Schlauch 14 und weiter dem Rohr 15 in den stromaufwärtigen Bereich des Steigrohres 30 presst.

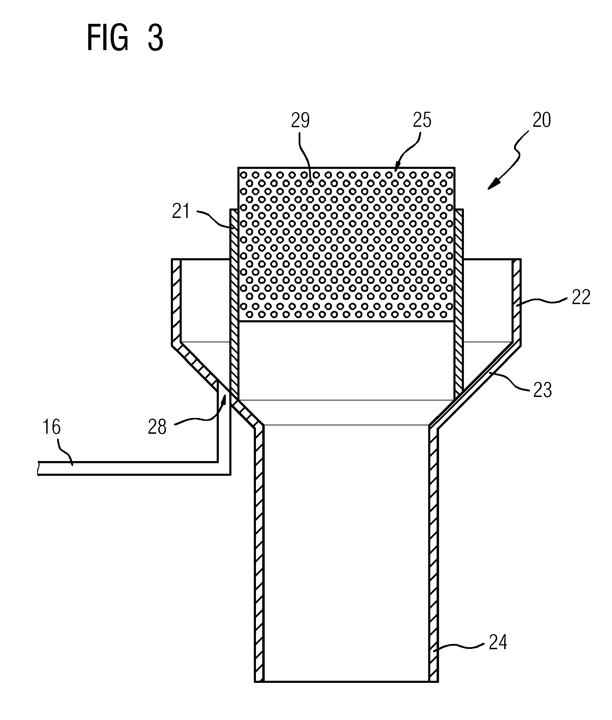

Der eingepresste Stickstoff 70 steigt im Steigrohr 30 in Durchströmungsrichtung 39 nach oben (reißt dabei - als Teil des Umwälzstroms - Medium (Minenwasser 10, behandeltes Minenwasser 17, Sandpartikel 41 bzw. Trägermaterial 41, Biomasse 45, Ausfällungen 42 sowie mit Biomasse 45 und Ausfällungen 42 verkrustetes Trägermaterial 43) im Steigrohr 30 mit nach oben bzw. fördert dadurch dieses Medium als Teil des Umwälzstrom 46 im Steigrohr 30 mit nach oben) und dringt - über eine dortige, d.h. am stromabwärtigen Ende 31 des Steigrohrs 30 befindliche - Abscheidvorrichtung 20, d.h. einem Schlammabsetztrichter 20 (vgl. Fig. 3) - in den Gasraum 4 des Reaktors 1 ein, von wo er durch die Membranpumpe 7 über das Abströmrohr 9 wieder aus dem Reaktor 1 abgezogen wird.

Dieser (Stickstoff-)Kreislauf bildet so eine - mittels der Membranpumpe betriebene - Mammutpumpe 6 aus, welche die Umwälzung 130 des Umwälzstroms 46 durch die Umwälzvorrichtung 60 erzeugt.

Am stromabwärtigen Ende 31 des Steigrohrs 30 ist, wie Fig.1 und Fig. 3 zeigen, diesem der - die Abscheidvorrichtung 20 bildende und in die Umwälzung 130 eingebundene - Schlammabsetztrichter 20 "übergestülpt", in welchen der Umwälzstrom 46 - vom Steigrohr 30 kommend bzw. von stromabwärtigen Ende 31 des Steigrohrs 30 kommend einströmt.

Dieser Schlammabsetztrichter 20 wird aus mehreren miteinander verbundenen - im Wesentlichen rohrförmigen - Rohrstücken 24, 23 und 22 gebildet, welche - zusammen - den Trichterhals 24 und den Trichtertopf 47 des Schlammabsetztrichters 20 ergeben.

Ein erstes - den Trichterhals 24 des Schlammabsetztrichters 20 bildendes - Rohrstück 24 weist einen über die gesamte Länge des Rohrstücks 24 konstanten Querschnitt auf.

Mit einem Ende mit dem Trichterhals 24 fest verbunden schließt sich daran ein zweites, den ersten Teil des Trichtertopfes 47 bildendes Rohrstück 23 an, welches im Wesentlichen kegelförmig ausgebildet ist. Dort angeordnet bzw. fest verbunden mit dem kegelförmigen Rohrstück 23 ist ein drittes, das zweite Teilstück des Trichterkopfes 47 bildendes, im Wesentlichen zylindrisches Rohrstück 22.

Wie Fig. 1 und Fig. 3 weiter zeigt, ist in dem Trichtertopf 47 des Schlammabsetztrichters 20 ein - in einem (Träger-) Rohrstück 21 eingepasster bzw. dort aufgenommener - Filter 25 angeordnet.

Dieser Filter 25 wird gebildet durch ein eine Vielzahl von Öffnungen 29 aufweisendes Gitterelement 25, welches an die Innenwand des Trägerrohrstücks 21 anschließt. Die Öffnungen 29 des Filters 25 weisen dabei jeweils eine - dem Trägermaterial 41, d.h. den Sandpartikel 41 für einen Biofilm 44 im Reaktor 1 angepasste - Größe von ca. 1 mm auf, wodurch das Trägermaterial 41, dessen Größe die der Öffnungen 29 übersteigt, vor einem Durchtritt durch den Filter 25 gehindert wird.

Der Filter 25 ist, wie weiter Fig. 1 und Fig. 3 zeigen, mit in etwas 2/3 seiner Höhe in dem Trägerrohrstück 21 aufgenommen; mit in etwa 1/3 seiner Höhe überragt der Filter 25 das Trägerrohrstück 21 - an dessen - in Horizontalrichtung gesehen - oberem Ende.

Mit seinem unteren Ende ist das Trägerrohrstück 21 - in etwa der halben Höhe des zweiten, den ersten Teil des Trichtertopfes 47 bildenden, kegelförmigen Rohrstücks 23 - dort strömungsdicht in dem zweiten, den ersten Teil des Trichtertopfes 47 bildenden Rohrstücks 23 aufgesetzt bzw. fest mit diesem dort verbunden.

Wie Fig. 1 und Fig. 3 weiter zeigen, weist das zweite, den ersten Teil des Trichtertopfes 47 bildende, kegelförmige Rohrstück 23 eine - abgedichtete - Öffnung 28 für einen dort eingesetzten Schlammabzug 16 auf, welcher in Form eines Abfluss-)Rohres 16 ausgebildet ist.

Über diesen Schlammabzug 16 kann durch den Filter 25 ausgefiltertes Material, d.h. durch die Öffnungen 29 des Filters 25 durchgetretenes und sich im Schlammabsetztrichter 20 bzw. in dessen Trichtertopf 47 abgesetztes Material (Schlamm) 26, d.h. ein Gemisch aus Minenwasser 10 bzw. 17, Biomasse 45 und Ausfällungen 42, dem Schlammabsetztrichter 20 - und weiter aus dem Reaktor 1 abgezogen werden (Schlammabtrennung 27).

Dazu wird, wie die Fig. 1 und Fig. 3 auch zeigen, der Schlammabzug 16 im Wesentlichen horizontal vom Inneren des Reaktors 1 - über eine Öffnung in dem Reaktorgehäuse 3 - nach außen geführt.

Der Schlammabsetztrichter 20 ist dem stromabwärtigen Ende 31 des Steigrohrs 30 derart "übergestülpt", dass dessen Trichterhals 24 - in etwa über dessen gesamter Höhe - das stromabwärtige Ende 31 des Steigrohres 30 koaxial umfasst, wobei sich zwischen dem "innenliegenden" Steigrohrende 31 und dem "außenliegenden" Trichterhals 47 des Schlammabsetztrichters 20 - im Bereich ihrer Umfassung - ein nach unten hin offener Zwischenraum ausbildet. Über diesen Zwischenraum - zwischen Steigrohr 30 und Trichterhals 47 - kann von dem Filter 25 zurückgehaltenes Medium, d.h. das Trägermaterial 41 für den Biofilm 44, nach unten hin (zurück zum Umwälzbett 40/Biofilm 44) austreten 58.

Pumpparameter der Membranpumpe 7, wie Pumprate bzw. -menge, Pumpgeschwindigkeit, Dauer und Häufigkeit u.ä., können bei der Membranpumpe 7 - über eine über eine (Steuer-)Leitung 64 mit der Membranpumpe 7 verbundene Kontrolleinrichtung (nicht dargestellt) - eingestellt werden, um so die Umwälzung 130 des Umwälzstroms 46, das Lösen 131 von am Trägermaterial 41 angelagertem Material 43 bzw. das Lösen 131 der Verkrustungen 43 (im Steigrohr 30) und das Abscheiden 132 vom Trägermaterial 41 und den Verkrustungen 43 bzw. der gelösten Biomasse 45 und den Ausfällungen 42 (in der Abscheidvorrichtung 20) gezielt zu regulieren bzw. zu steuern.

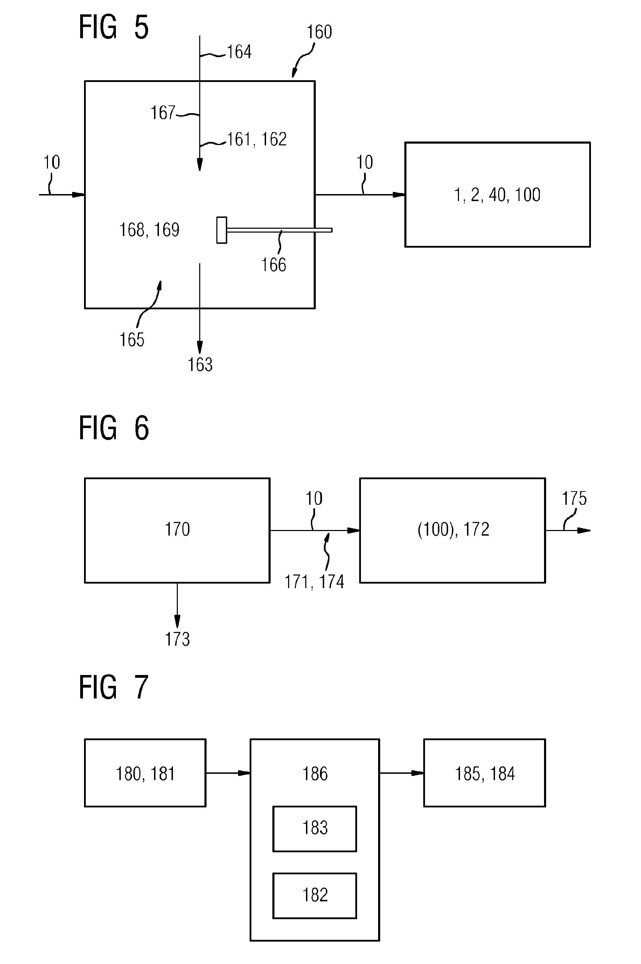

Über verschiedene Höhen im Reaktorraum 2 verteilt sind mehrere Sensoren 63 angeordnet, welche über Leitungen 64 - ebenfalls - mit der Kontrolleinrichtung verbunden sind. Mittels dieser Sensoren 63 werden - an den jeweiligen Positionen im Reaktorraum 2 - verschieden Prozessparameter, wie ph-Wert, Temperatur, Druck, Sauerstoffkonzentration und Redoxpotenzial, Substrat- und Nährstoffkonzentrationen, gemessen 180 (vgl. FIG. 7, 180), was eine nahezu vollständige räumliche Information über den Prozess bzw. über die Prozessbedingungen im Reaktor 1 liefert, und so - anhand dieser Information - die biologischen bzw. Prozessvorgänge im Reaktor 1 kontrolliert sowie damit auch die Umwälzung 130 - und darüber auch die Performance des Reaktors 1 - gesteuert.

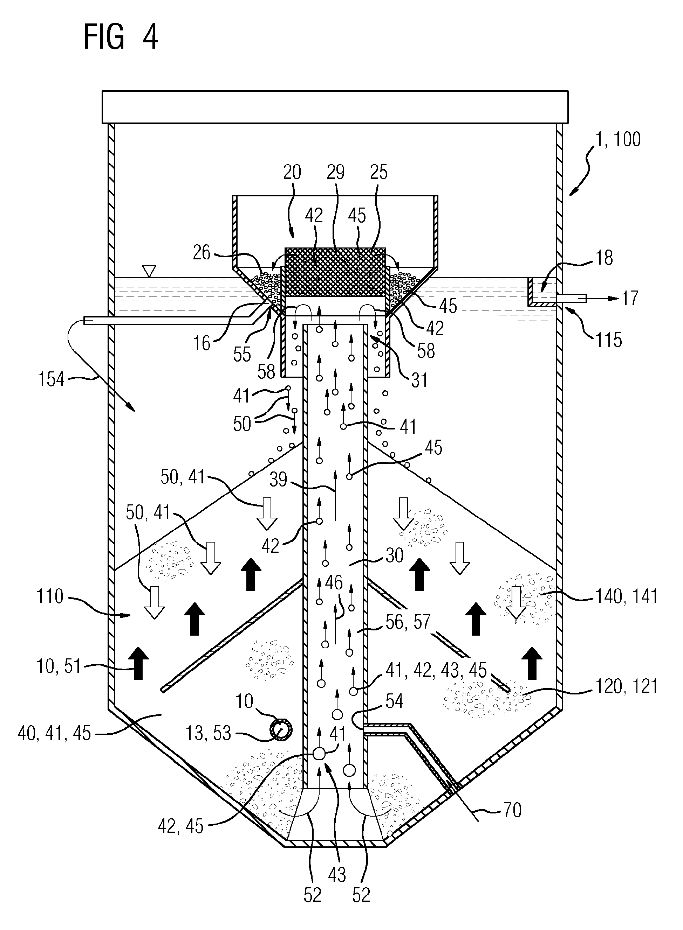

Fig. 4 zeigt den Reaktor 1 in vereinfachter Darstellung, verdeutlicht aber die im Reaktor 1, insbesondere bei der Reinigung 100 des zu behandelnden Minenwassers 10 sowie der Umwälzung 130 des Sandbetts 40 und der Umwälzung 130 des Umwälzstroms 46, ablaufenden Vorgänge.

Wie FIG 4 zeigt, befindet sich im unteren Bereich des Reaktors 1 das - periodisch umgewälzte - Umwälzbett 40 aus Trägermaterial 41 und dort immobilisierter Biomasse 45.

Das zu behandelnde Minenwasser 10 tritt - zur Reinigung - unter Druck über das Rohr 13 in den unteren Bereich des Reaktors 1 ein 53 - und steigt von dort nach oben 51, wobei es das Umwälzbett 40 durchströmt und dabei die Biomasse 45 kontaktiert 110.

Hierbei - bei einer Verweildauer des zu behandelnden Minenwassers 10 im Umwälzbett 40 von ca. 2 h - erfolgt - bei der Durchströmung und Kontaktierung 110 - die (biologische) Reinigung bzw. Behandlung des sulfat- und schwermetallhaltigen Minenwassers 10, 100.

Die Reinigungswirkung beruht auf der biologischen Reduktion von Sulfat bzw. Metallen durch die Biomasse 45, 120, wobei Metallsulfid, Metallcarbonat, Metalloxid oder Hydroxid, Metallphosphat 42, d.h. die Fällungsprodukte 42, ausgefällt werden 120.

Die Ausfällungen 42 werden im Sandbett 40 zurückgehalten, wobei diese das Trägermaterial 41 dort verkrusten 121, das Umwälzbett 40 verstopfen und - wie insbesondere auch der über die Zeit im Umwälzbett 40 anwachsende Biofilm 44 - unerwünschte bevorzugte Fließwege im Umwälzbett 40 ausbilden.

Das behandelte/gereinigte Abwasser 17 wird mittels des Ablaufs 18 aus dem Reaktor 1 abgezogen 115.

Um der unerwünschten Ausbildung von bevorzugten Fließwegen, insbesondere durch das Anwachsen des Biofilms 44, im Umwälzbett 40 entgegenzuwirken, d.h. um die Dicke des Biofilms 44 zu kontrollieren und gegebenenfalls zu reduzieren, wird das Umwälzbett 40 periodisch, beispielsweise einmal pro Woche, umgewälzt 130.

Der Antrieb der Umwälzung 130 erfolgt mittels des - durch die Membranpumpe 7 geförderten - Stickstoffstroms 70, welcher als Mammutpumpe 6 wirkt.

Hierzu wird der Stickstoff 70 - über das Rohr 15 - im Bereich des stromaufwärtigen (unteren) Endes des Steigrohres 30 in dieses gepresst 54. Der Stickstoff 70 strömt im Steigrohr 30 in der Durchströmungsrichtung nach oben, wobei er - über das offene, stromaufwärtige Ende des Steigrohrs 30 - Minenwasser 10, Trägermaterial 41 bzw. verkrustetes Trägermaterial 43, Ausfällungen 42 und Biomasse 45, d.h. den Umwälzstrom 46, in das Steigrohr 30 zieht 52 und diesen durch das Steigrohr 30 in der Durchströmungsrichtung 39 nach oben fördert.

Während der Durchströmung des Umwälzstroms 46 durch das Steigrohr 30 nach oben entstehen im Umwälzstrom 46 Turbolenzen 56, wodurch sich das verkrustete Trägermaterial 43 aneinander reibt. Hierdurch lösen 57 sich die Verkrustungen, d.h. die Ausfällungen 42 und die Biomasse 45, von dem Trägermaterial 41 - und werden, wie FIG 4 zeigt, separiert in der Durchströmungsrichtung 39 im Steigrohr weiter nach oben gefördert.

Die Strömung bzw. der Umwälzstrom 46 trifft am stromaufwärtigen, oberen Ende 31 des Steigrohres 30 auf den Schlammabsetztrichter 20. An den Gitteröffnungen 29 des Gitterelements 25 des Schlammabsetztrichters 20 wird das Trägermaterial 41 - aufgrund seiner die Gitteröffnungen 29 übersteigenden Größe - zurückgehalten 58, wohingegen die kleinere Biomasse 45 und die Ausfällungen 42 - durch das weiter nach oben strömende Minenwasser 10 - durch die Gitteröffnungen 29 des Gitterelements 25 durchgespült werden.

Das - von den Verkrustungen 43 befreite - Trägermaterial 41 sinkt - unter Schwerkrafteinfluss in dem Durchlass zwischen dem stromabwärtigen Ende 31 des Steigrohrs 30 und dem dort übergestülptem Schlammabsetztrichter 20 nach unten 50 und setzt sich wieder - befreit von der Biomasse und den Ausfällungen - als gereinigtes Trägermaterial 41 im Umwälzbett 40 ab.

Die ausgefilterte Biomasse 45, die ausgefilterten Ausfällungen setzen sich - zusammen mit dem Minenwasser 10 - nach Austritt aus dem Gitterelement 25 als Schlamm 26 in dem Schlammabsetztrichter 20 ab und werden von dort über den Schlammabzug 16 aus dem Reaktor 1 abgezogen 55.

Ein Teil der ausgefilterten Biomasse 45 wird dem Biofilm 44 im Reaktor 1 wieder zugegeben 154, wodurch eine Unterstützung und schnelle Bildung des Biofilms 44 im Umwälzbett 40 erreicht wird bzw. die Biofilmbildung gesteuert werden kann.

Über die fortwährende Umwälzung 130 setzt sich - kontinuierlich - um die immobilisierte Biomasse 45 gereinigtes Trägermaterial 41 an der Oberfläche des Umwälzbettes 40 ab, wohingegen am gegenüberliegenden unteren Ende des Umwälzbettes 40 das verkrustete Trägermaterial 43 ständig abgezogen wird.

Anschaulich ausgedrückt, das um die Biomasse 45 und die Ausfällungen 42 gereinigte Trägermaterial 41 durchwandert so das Umwälzbett 40 von oben nach unten 50, wodurch sich das Umwälzbett 40 erneuert 141. Dadurch lösen sich die Verstopfungen und die unerwünschten bevorzugten Fließwege im Umwälzbett 40 auf.

Die Biofilmerneuerung kann so über die Wanderungsgeschwindigkeit des Umwälzbetts 40 reguliert werden 140. Auch die Dauer der Umwälzung beeinflusst die Biofilmerneuerung.

Kurz, der Biofilm 45 wird über die Umwälzung 130 kontrolliert bzw. reguliert 140, wobei die Pumprate der Membranpumpe 7 sowie der Umwälzgeschwindigkeit der Membranpumpe 7 reguliert werden können.

FIG 5 zeigt schematisch eine - der Behandlung 100 des Minenwassers 10 im Reaktor 1 vorgeschaltete (außerhalb des Reaktors 1 durchgeführte) - biologische Oxidation 160 des Minenwassers 10 (vorgeschaltete biologische Oxidationsstufe 160).

Bei der biologischen Oxidation 160 bzw. der vorgeschalteten biologischen Oxidationsstufe 160 werden in dem Minenwasser 10 enthaltene Metalle, im Speziellen zweiwertiges Eisen, unter Verwendung von Mikroorganismen 169 oxidiert bzw. zu Eisen(III) oxidiert 161. Anschließend wird das oxidierte Metall bzw. das Eisen(III) als feste Metallverbindung bzw. als feste Eisenverbindung, beispielsweise als Eisenoxide oder Eisenhydroxide, ausgefällt 162, wodurch die Eisenkonzentration im Minenwasser 10 deutlich gesenkt wird, und das derart vorbehandelte Minenwasser 10 weiter dann dem Reaktor 1 zur weiteren Behandlung 100 zugeführt wird.

Die abgetrennte/ausgefällte feste Metall-/Eisenverbindung wird einer rohstofflichen Nutzung oder einer Deponierung zugeführt 163.

Wie FIG 5 verdeutlicht, erfolgt die - unter Verwendung der Mikroorganismen 169 durchgeführte - biologische Oxidation 161 in einem Oxidationsbecken 165 - bei einem dortig eingestellten pH-Wert von ca. 3 - , in welches das Minenwasser 10 zunächst einströmt, dort - bei einer Verweilzeit im Oxidationsbecken von ca. 8 h - der biologischen Oxidation 161 mittels der Mikroorganismen 169 unterzogen wird, wobei das bei der biologischen Oxidation 161 entstehende oxidierte Metall bzw. Eisen aus dem Minenwasser 10 abgetrennt 162 wird, und anschließend das - um das Metall reduzierte - Minenwasser 10 dem Reaktor 1 zur dortigen Behandlung 100 zugeführt wird.

In dem Oxidationsbecken 165 sind die Mikroorganismen 169 auf Aufwuchskörper 168, hier Kunststoffkörper 169, angesiedelt und bilden dort Biomasse 169 aus. Eine Rührvorrichtung 166 im Oxidationsbecken 165 bewirkt eine verbesserte Kontaktierung des Minenwassers 10 mit den Mikroorganismen 169 bzw. der Biomasse - und fördert dadurch die biologische Oxidation 161.

Mittels eines Gebläses 167 wird das Oxidationsbecken 165 bei der biologischen Oxidation 161 mit Sauerstoff 164 belüftet.

Durch diese vorgeschaltete biologische Oxidationsstufe 160 wird die Metall-/Eisenkonzentration im Minenwasser 10 vor dessen Eintritt in den Reaktorraum 2 des Reaktors 1 deutlich gesenkt, wodurch es dort zu wesentlich weniger Verblockungen des Biofilms 44 auf dem Trägermaterial 41 und weniger Verblockungen von Fließwegen im Umwälzbett 40 kommt. Dadurch wird auch die unerwünschte Ausbildung von bevorzugten Fließwegen behindert.

Dies alles führt wiederum zu einem deutlich höheren Stoffaustausch zwischen dem Biofilm 44 und dem zu behandelndem Abwasser 10 im Reaktor 1 und damit zu einer verbesserten Reinigungsleistung im Reaktorraum 2.

FIG 6 zeigt schematisch ein Verfahren zur Behandlung der Minenwässer 10, bei dem bei dem Minenwasser 10 zunächst ein Membranverfahren 170, hier eine Nanofiltration 170, durchgeführt wird. Das bei der Nanofiltration 170 - neben dem gereinigten Abwasser 173 auch - erzeugte Konzentrat 174 wird anschließend einem Bioreaktor zugeführt 171, wobei bei dem Konzentrat eine biologische Sulfatreduktion 172 angewandt wird, wodurch - weiter - gereinigtes Minenwasser 175 (aus dem Konzentrat 174) erzeugt wird.

Diese Kombination von - vorgeschalteter - Membranfiltration bzw. Nanofiltration 170 und - nachfolgend am Konzentrat 174 durchgeführter - allgemeiner biologischen Sulfatreduktion 172 erreicht eine Verringerung der - bei der nachgeschalteten biologischen Behandlung 172 - zu behandelnden Wassermenge, wodurch die für die biologische Sulfatreduktion 172 einsetzbaren Bioreaktoren kompakt gebaut sein können, was Bau- und Investitionskosten spart.

Auch können hierdurch die Kosten der Membran- bzw. Nanofiltrationsstufe 170 begrenzt werden, da dort immer eine gewisse Restmenge an Konzentrat 174 übrigbleibt, das wirtschaftlich nicht weiter durch diese Filtrationstechnik aufgearbeitet werden kann.

Eine kostenintensive Deponierung oder Weiterbehandlung dieses Konzentrats 174 durch andere Technologien wäre notwendig.

Eine - ökologisch und ökonomisch sinnvolle - Aufbereitung dieses Konzentrats 174 wird so dann durch die nachgeschaltete biologische Sulfatreduktion 172 erreicht, wobei es zu einer weiteren Reduktion der Sulfat- und Metallkonzentrationen im behandelten Minenwasser kommt.

FIG 7 verdeutlicht schematisch eine über die Kontrolle der mikrobiologischen Lebensgemeinschaft, d.h. der Mikroorganismen, und deren Prozessbedingungen im Reaktor 1 gesteuerte Behandlung 100 des Minenwassers 10 im Reaktor 1.

Die Zusammensetzung der mikrobiologischen Lebensgemeinschaft im Reaktor 1 spielt dabei eine entscheidende Rolle für die im Reaktor 1 ablaufenden biologischen Prozesse, d.h. die Reduktionsreaktionen, wie die biologische Sulfat-/Metallreduktion, bei dem Minenwasser 10. Beispielsweise nimmt sie auch Einfluss auf die Geschwindigkeit der ablaufenden Sulfat-/Metallreduktion im Reaktor 1, was wiederum für eine effektive und effiziente kommerzielle Nutzung eines biologischen Prozesses bzw. der biologischen Sulfatreduktion und/oder eines solchen Bioreaktors 1 von entscheidender Bedeutung ist.

Diese biotechnologischen Prozesse erfordern eine genaue Kontrolle der Reaktionsbedingungen (im Reaktor 1), da die genutzten Katalysatoren, d.h. die Biomasse 45 bzw. die Mikroorganismen 45, ein enges Optimum besitzen. Dies sind insbesondere Temperatur und pH-Wert. Darüber hinaus spielen aber auch Nährstoffe, Substrate und Spurenelemente bzw. deren Konzentrationen eine Rolle.

Erschwerend bei der biologischen Sulfatreduktion von Minenwässern 10 ist die zeitlich inhomogene Natur der zulaufenden Minenwässer 10, deren Sulfat- und Schwermetallfracht - je nach Bedingungen im Bergbau - massiven Schwankungen unterliegt. Gleichzeitig handelt es sich bei dem Minenwasser 10 um kein steriles Medium und der Eintrag von - die Reduktionsprozesse fördernden, wie aber auch dazu konkurrierende - Mikroorganismen ist möglich und anzunehmen.

Es ist daher sicherzustellen, dass die mikrobiologische Lebensgemeinschaft und deren Stoffwechsel dem optimalen Zustand entsprechen. Der optimale Zustand ist dabei allgemein so definiert, dass Nährstoffe, wie das Methanol, optimal genutzt und die für die Reduktionsprozesse vorgesehenen mikrobiologischen Stämme die gewünschten Reaktionen katalysieren.

Um eine Kontrolle dieses optimalen Zustands sicherzustellen, sind, wie FIG 1 zeigt, - über verschiedene Höhen im Reaktorraum 2 verteilt - mehrere Sensoren 63 angeordnet, welche - an den jeweiligen Positionen im Reaktorraum 2 - verschiedene Prozessparameter, wie ph-Wert, Temperatur, Druck, Sauerstoffkonzentration und Redoxpotenzial, Substrat- und Nährstoffkonzentrationen, - kontinuierlich über die Zeit - messen 180, 181.

Hierdurch erhält die mit den Sensoren 63 verbundene Kontrolleinrichtung, welche u.a. auch die Umwälzung 130 sowie die Zugabe von Nährstoffen wie auch eine Temperatur-, Druck-, Sauerstoffkonzentrations- und Redoxpotenzieleinstellung (mittels entsprechender Aktuatoren) kontrolliert, regelt und steuert 185, eine nahezu vollständige räumliche und auch zeitliche Information über die Prozess- bzw. über die Prozessbedingungen im Reaktor 1.

So liegen in der Kontrolleinrichtung die zeitlichen - und auch räumlichen - Zustandswerte von Temperatur, Sauerstoffkonzentration, pH-Wert, Nährstoffe, Substrate und Spurenelemente sowie deren jeweilige Konzentrationen im Reaktor 1 vor.

In der Kontrolleinrichtung werden, wie FIG 7 auch verdeutlicht, diese räumlichen und zeitlichen Prozessparameter für die Kontrolle, Steuerung und Regelung 185 bzw. Einstellung 185 des gewünschten Zustandes der mikrobiologischen Lebensgemeinschaft in dem Reaktor 1 sowie der Kontrolle der Umwälzung bzw. des Reaktors 1 ausgewertet 186.

Die Auswertung 186 und Kontrolle 185 des gewünschten Zustandes der mikrobiologischen Lebensgemeinschaft erfolgt, wie FIG 7 zeigt, einerseits über die Überwachung 183 der - mittels der Sensorik 63 gemessenen - extrazellulären Carbonsäure im Reaktor 1.

Dabei wird hier nicht der Zusammenhang zwischen den einzelnen beteiligten Mikroorganismen und den Carbonsäuren aufgeklärt, sondern die erkennbaren Muster mithilfe einer statistischen Auswertung (multivariate Datenanalyse, wie z.B. Hauptkomponentenanalyse) gewünschten Zuständen des Reaktors 1 zugeordnet 183.

Diese Information wird dann zur Regelung 185 des Reaktors bzw. der Einstellung 185 des optimalen Zustands der biologischen Lebensgemeinschaft im Reaktor 1 eingesetzt.

Daneben erfolgt, wie FIG 7 auch verdeutlicht, die Auswertung 186 und Kontrolle 185 über die Überwachung der im Reaktor 1 miteinander konkurrierenden Mikroorganismen 182.

Hierbei wird einerseits auf die mittels der Sensorik 63 gemessenen Parameter zurückgegriffen, von welchen bekannt ist, dass sie Einfluss auf eine erfolgreiche ablaufende biologische Reduktion haben, wie der pH-Wert, das Redoxpotenzial, die Sulfatkonzentration und die Substratkonzentration, andererseits auf die durch die Sensorik 63 gemessenen Parameter der Abbauprodukte der mit den sulfatreduzierenden Mikroorganismen konkurrierenden Mikroorganismen, wie organische Carbonsäuren, beispielsweise Essigsäure oder Buttersäure - als Abbauprodukte fermentierender Mikroorganismen, und Methan - als Endprodukt methanogener Mikroorganismen 180, 181.

Diese Information wird dann zur Regulierung der Konkurrenz zwischen den unterschiedlichen, d.h. fördernden und hemmenden (konkurrierenden), Mikroorganismen im Reaktor 1 und damit zur Regelung 185 des Reaktors bzw. Einstellung/Regulierung des optimalen Zustands der biologischen Lebensgemeinschaft im Reaktor 1 185 eingesetzt.

Diese - auf die Beeinflussung der Konkurrenzsituation im Reaktor 1 gerichtete Steuerung 184 - erfolgt - aktionsseitig - durch Regulierung 184 der Aufenthaltsdauer des Abwassers 10 im Reaktorraum 2 184, wobei eine kurze Aufenthaltsdauer des zu behandelnden Minenwasser 10 im Reaktor 1 zu einer selektiven Anreicherung von sulfatreduzierenden Mikroorganismen im Biofilm 44 auf dem Trägermaterial 41 führt. Die dazu konkurrierenden Mikroorganismen, wie zum Beispiel methanproduzierende Mikroorganismen, werden dadurch in ihrer Vermehrung gehemmt.

Weitere Steuerungsmöglichkeiten 184 zur Beeinflussung der Konkurrenzsituation zwischen den verschiedenen mikrobiologischen Spezien sind die Regulierung 184 des ph-Werts des Abwassers 10 im Reaktorraum 2 in einem Bereich, welcher nicht optimal für die unerwünschten Mikroorganismen, ist oder eine kurzzeitige Sauerstoffdosierung 184 in dem Reaktorraum 2, wobei die sulfatreduzierenden Mikroorganismen durch geringe Sauerstoff-Partialdrücke weniger gehemmt werden als z.B. Methanogene.

Weiter kann als Steuerungsmöglichkeit 184 der Konkurrenzsituation das Verhältnis von Substratkonzentration zu Sulfatkonzentration gesteuert werden 184.

Obwohl die Erfindung im Detail durch die bevorzugten Ausführungsbeispiele näher illustriert und beschrieben wurde, so ist die Erfindung nicht durch die offenbarten Beispiele eingeschränkt und andere Variationen können vom Fachmann hieraus abgeleitet werden, ohne den Schutzumfang der Erfindung zu verlassen.

## Patentansprüche

1. Vorrichtung (1) zur Behandlung von Abwässern, insbesondere von sulfat- und/oder schwermetallhaltigen Minenwässern, mit
- einem Reaktorraum (2),
- einer im Wesentlichen in dem Reaktorraum (2) angeordneten Umwälzvorrichtung (60) zu einer, insbesondere periodischen, Umwälzung eines Umwälzstroms (46) aus einem sich in dem Reaktorraum (2) befindenden Abwasser (10) sowie einem in dem Reaktorraum (2) angeordneten Trägermaterial (41), auf welchem Biomasse (45) eines Biofilms (44) immobilisiert ist, und
- einer in dem Reaktorraum (2) angeordneten, von dem Umwälzstrom (46) durchströmbaren Abscheidvorrichtung (20), welche eine Filtervorrichtung (25) aufweist, welche an das Trägermaterial (41) angepasste Öffnungen (29) aufweist, wobei aus dem Umwälzstrom (46) die während der Umwälzung des Umwälzstroms (46) von dem Trägermaterial (42) gelöste Biomasse (45) aus dem Umwälzstrom (46) ausscheidbar ist.

2. Vorrichtung (1) zur Behandlung von Abwässern nach mindestens einem der voranstehenden Ansprüche,
**dadurch gekennzeichnet, dass** die Filtervorrichtung (25) ein die Öffnungen (29) aufweisendes Gitterelement (25) oder ein die Öffnungen (29) aufweisendes Lochblech (25) aufweist, wobei die Öffnungen (29) bzw. die Löcher (29) derart bemessen sind, dass das Trägermaterial (41) vor einem Durchtritt durch das Gitterelement (25) bzw. Lochblech (25) zurückgehalten wird.

3. Vorrichtung (1) zur Behandlung von Abwässern nach mindestens einem der voranstehenden Ansprüche,
**dadurch gekennzeichnet, dass** die Abscheidvorrichtung (20) einen von dem Umwälzstrom (46) in einer Durchströmungsrichtung (39) durchströmbaren Schlammabsetztrichter (20) mit einem Trichterhals (24) und einem Trichtertopf (47) aufweist, wobei an dem Trichtertopf (47) die Filtervorrichtung (25) angeordnet ist, und/oder dass die Umwälzvorrichtung (60) ein von dem Umwälzstrom (46) in der Durchströmungsrichtung (39) durchströmbares Steigrohr (30) aufweist, auf welchem an dessen stromabwärtigem Ende (31) ein an seiner Außenseite profilierter Rohrausatz (32) aufgesteckt ist.

4. Vorrichtung (1) zur Behandlung von Abwässern nach mindestens dem voranstehenden Anspruch,
**dadurch gekennzeichnet, dass** der Trichterhals (24) das stromabwärtige Ende (31) des Steigrohres (30) koaxial umfasst.

5. Vorrichtung (1) zur Behandlung von Abwässern nach mindestens einem der voranstehenden Ansprüche,
**dadurch gekennzeichnet, dass** das Steigrohr (30) einen auf das Steigrohr (30) aufgesteckten Trichter (35) aufweist, wobei der Trichter (35) derart auf das Steigrohr (30) aufgesteckt ist, dass eine Öffnung (48) eines Trichtertopfes (47) des Trichters (35) entgegen der Durchströmungsrichtung (39) des Steigrohrs (30) geöffnet ist.

6. Vorrichtung (1) zur Behandlung von Abwässern nach mindestens einem der voranstehenden Ansprüche,
**dadurch gekennzeichnet, dass** die Umwälzvorrichtung (60) eine Vorrichtung nach einem Prinzip einer Mammutpumpe (6) aufweist, welche insbesondere mittels einer Membranpumpe (7) betrieben wird.

7. Vorrichtung (1) zur Behandlung von Abwässern nach mindestens dem voranstehenden Anspruch,
**dadurch gekennzeichnet, dass** die Membranpumpe (7) mit einem Gas (70), insbesondere Stickstoff oder Kohlendioxid (70), oder einer Flüssigkeit (70), insbesondere dem Abwasser (10), betreibbar ist, wobei insbesondere das Gas (70) oder die Flüssigkeit (70) in einem Bereich am stromaufwärtigen Ende des Steigrohres (30) in das Steigrohr (31) einpressbar ist.

8. Vorrichtung (1) zur Behandlung von Abwässern nach mindestens einem der beiden voranstehenden Ansprüche,
**dadurch gekennzeichnet, dass** über eine Pumprate und/oder Pumpfrequenz der Vorrichtung nach dem Prinzip der Mammutpumpe (6) und/oder der Membranpumpe (7) die Umwälzung des Trägermaterials (41 bzw. 42) kontrollierbar ist (Regulierung Wanderungsgeschwindigkeit des Umwälzbetts (40)).

9. Vorrichtung (1) zur Behandlung von Abwässern nach mindestens einem der voranstehenden Ansprüche,
gekennzeichnet mit einem am Reaktorraum (2) angeordneten Zufluss (11) für einen Zufluss des Abwassers (10) in den Reaktorraum (2) und einem am Reaktorraum (2) angeordneten Ablauf (18) für einen Ablauf von in der Vorrichtung (1) behandeltem Abwasser (17) aus dem Reaktorraum (2), wobei insbesondere der Ablauf (18) einen Überströmrand (59) aufweist, über welchen das behandelte Abwasser (17) abziehbar ist.

10. Vorrichtung (1) zur Behandlung von Abwässern nach mindestens einem der voranstehenden Ansprüche,
verwendet bei einem Biobettreaktor mit periodisch umgewälztem Biobett.

11. Vorrichtung (1) zur Behandlung von Abwässern nach mindestens einem der voranstehenden Ansprüche,
eingesetzt zu einer periodischen Umwälzung des Umwälzstroms (46).

12. Vorrichtung (1) zur Behandlung von Abwässern nach mindestens einem der voranstehenden Ansprüche,
eingesetzt zur Behandlung und/oder Reinigung von Minenwässer aus Bergbaubetrieben, insbesondere zur Aufarbeitung von "saurem Minenablauf" (acid mine drainage AMD) oder "saurem Gesteinsablauf" (acid rock drainage ARD) oder zur Aufarbeitung von mit anderen Verfahren, wie beispielsweise einer Membranfiltration, aufkonzentrierten Minenwässern, oder von Abwässern aus einer metallverarbeitenden Industrie.

13. Verfahren (100) zur Behandlung von Abwässern, insbesondere von sulfat- und/oder schwermetallhaltigen Minenwässern, in einer Vorrichtung zur Behandlung von Abwässern mit einem Reaktorraum (2), einer im Wesentlichen in dem Reaktorraum (2) angeordneten Umwälzvorrichtung (60) zu einer, insbesondere periodischen, Umwälzung eines Umwälzstroms (46) aus einem sich in dem Reaktorraum (2) befindenden Abwasser (10) sowie einem in dem Reaktorraum (2) angeordneten Trägermaterial (41), auf welchem Biomasse (45) eines Biofilms (44) immobilisiert ist, und einer in dem Reaktorraum (2) angeordneten, von dem Umwälzstrom (46) durchströmbaren Abscheidvorrichtung (20), insbesondere in einer Vorrichtung gemäß einem der voranstehenden Vorrichtungsansprüchen,
bei dem
- der Umwälzstrom (48) unter Verwendung der Umwälzvorrichtung (60), insbesondere periodisch, in dem Reaktorraum (2) umgewälzt wird,
- bei der, insbesonderen periodischen, Umwälzung des Umwälzstroms (46) die auf dem Trägermaterial (41) immbolisierte Biomasse (45) von dem Trägermaterial (41) gelöst wird und unter Verwendung der Abscheidvorrichtung (20) die gelöste Biomasse (45) aus dem Abwasser (10) abgeschieden (132) wird,
- wobei mittels der Umwälzung (130) der Biofilm (44), insbesondere ein Anwachsen der auf dem Trägermaterial (41) immobilisierten Biomasse (45) des Biofilms (44) und/oder eine Dicke des Biofilms (44), kontrolliert wird (140).

14. Verfahren (100) zur Behandlung von Abwässern nach mindestens einem der voranstehenden Ansprüchen,
**dadurch gekennzeichnet, dass** das Trägermaterial (41) zu einem Start der Vorrichtung, insbesondere der Vorrichtung (1) nach mindestens einem der voranstehenden Vorrichtungsansprüche, durch ein Animpfen mit Mikroorganismen besiedelt wird (mikrobielle Startkultur) (152) und/oder dass die mikrobielle Startkultur durch ein Auffiltern von bereits im Abwasser (10) enthaltenen Mikroorganismen auf das Trägermaterial (10) hergestellt wird (153).

15. Verfahren (100) zur Behandlung von Abwässern nach mindestens einem der voranstehenden Ansprüchen,
**dadurch gekennzeichnet, dass** die aus dem Abwasser (10) ausgeschiedene Biomasse (45) zumindest zum Teil dem Abwasser (10) wieder zugeführt wird (154).

16. Verfahren (100) zur Behandlung von Abwässern nach mindestens einem der voranstehenden Ansprüchen,
**dadurch gekennzeichnet, dass** ein Redoxpotenzial des Abwassers (10) verringert wird (150), insbesondere unter Zugabe von reduzierten Schwefelverbindungen, wie Sulfide, zu dem Abwasser (10).

17. Verfahren (100) zur Behandlung von Abwässern nach mindestens einem der voranstehenden Ansprüchen,
**dadurch gekennzeichnet, dass** dem Abwasser (10) Nährstoffe, insbesondere eine Kohlenstoff-Quelle, zugegeben werden, welche an Mikroorganismen, welche den Biofilm (44) erzeugen, angepasst sind (151).

18. Verfahren (100) zur Behandlung von Abwässern nach mindestens einem der voranstehenden Ansprüche,
**dadurch gekennzeichnet, dass** eine Verweildauer von dem Abwasser (10) in einem das Trägermaterial (41) und den an dem Trägermaterial (41) angewachsenen Biofilm (44) aufweisenden Umwälzbett (40) in einem Bereich von ungefähr 30 min bis 5 Stunden, insbesondere in einem Bereich von ungefähr 1 Stunde bis 3 Stunden, gehalten wird.

19. Verfahren (100) zur Behandlung von Abwässern nach mindestens einem der voranstehenden Ansprüche,
**dadurch gekennzeichnet, dass** bei dem behandelten Abwasser (17) eine Nachbehandlung, insbesondere eine biologische Oxidation oder ein Ausstrippen von Schwefelwasserstoff, durchgeführt wird.

20. Verfahren (100) zur Behandlung von Abwässern nach mindestens dem voranstehenden Anspruch,
**dadurch gekennzeichnet, dass** der ausgestrippte Schwefelwasserstoff als Fällungschemikalie weiterverwendet wird.

21. Verfahren (100) zur Behandlung von Abwässern nach mindestens einem der voranstehenden Ansprüche,
**dadurch gekennzeichnet, dass** bei dem Abwasser (10) vor der Behandlung des Abwassers (10) in dem Reaktorraum (2) eine biologische Oxidation durchgeführt wird (160).

22. Verfahren (100) zur Behandlung von Abwässern nach mindestens dem voranstehenden Anspruch,
**dadurch gekennzeichnet, dass** bei der biologischen Oxidation (160) in dem Abwasser (10) enthaltene Metalle, insbesondere Eisen, im Speziellen zweiwertiges Eisen, oxidiert werden und aus dem Abwasser abgetrennt werden (162)

23. Verfahren (100) zur Behandlung von Abwässern nach mindestens dem voranstehenden Anspruch,
**dadurch gekennzeichnet, dass** das oxidierte Eisen in Form von Schwertmannit von dem Abwasser (10) abgetrennt (162) wird.

24. Verfahren (100) zur Behandlung von Abwässern nach mindestens einem der drei voranstehenden Ansprüche,
**dadurch gekennzeichnet, dass** die biologische Oxidation (160) bei einem pH-Wert von ungefähr 3 bei dem Abwasser (10) durchgeführt wird und/oder bei dem die biologische Oxidation (160) jeweils bei unterschiedlich eingestellten pH-Werten des Abwassers (10) erfolgt, wodurch bei einem jeweils eingestellten pH-Wert im Abwasser (10) gezielt ein bestimmtes Metall im Abwasser (10) oxidiert wird.

25. Verfahren (100) zur Behandlung von Abwässern nach mindestens einem der voranstehenden Ansprüche,
**dadurch gekennzeichnet, dass** bei dem Abwasser (10) vor der Behandlung des Abwassers (10) in dem Reaktorraum (2) eine Membranfiltration (170), insbesondere eine Nanofiltration (170), durchgeführt wird (170), wobei ein durch die Membranfiltration, insbesondere die Nanofiltration, erzeugtes Konzentrat (174) als das Abwasser (10) dem Reaktorraum (2), insbesondere der Vorrichtung (1) gemäß einem der voranstehenden Vorrichtungsansprüche, zugeführt wird (171).

26. Verfahren (100) zur Behandlung von Abwässern nach mindestens einem der voranstehenden Ansprüche,
**dadurch gekennzeichnet, dass** bei dem im Reaktorraum (2) befindlichen Abwasser (10) und/oder bei dem behandelten Abwasser (17) ein physikalischer und/oder chemischer Parameter, insbesondere ein pH-Wert, eine Temperatur, ein Redoxpotential, eine Sulfatkonzentration, eine Substratkonzentration (Elektronendonatorkonzentration), eine Nährstoffkonzentration, eine Konzentration von durch die Mikroorganismen (44) erzeugten Abbauprodukten und/oder ein Umfang der Mikroorganismen (44), oder mehrere solche physikalische und/oder chemische Parameter bestimmt, insbesondere kontinuierlich gemessen, wird bzw. werden (180).

27. Verfahren (100) zur Behandlung von Abwässern nach mindestens dem voranstehenden Anspruch,
**dadurch gekennzeichnet, dass** der oder die mehreren physikalischen und/oder chemische Parameter an oder jeweils an unterschiedlichen Positionen in dem Reaktorraum (2) bestimmt, insbesondere kontinuierlich gemessen, wird bzw. werden (180).

28. Verfahren (100) zur Behandlung von Abwässern nach mindestens einem der beiden voranstehenden Ansprüche,
**dadurch gekennzeichnet, dass** unter Verwendung des oder der bestimmten, insbesondere kontinuierlich gemessenen, physikalischen und/oder chemischen Parameter die Behandlung des Abwassers (10) in dem Reaktorraum (2), insbesondere durch Steuerung der Umwälzung (130), gesteuert wird.

29. Verfahren (100) zur Behandlung von Abwässern nach mindestens einem der voranstehenden Ansprüche,
**dadurch gekennzeichnet, dass** bei dem im Reaktorraum (2) befindlichen Abwasser (2) und/oder bei dem behandelten Abwasser (17) ein Umfang von mit den Mikroorganismen (44) konkurrierenden (Mikro-)Organismen und/oder von Abbauprodukten (81) von den mit den Mikroorganismen (44) konkurrierenden (Mikro-) Organismen, insbesondere eine Konzentration von organischen Carbonsäuren (Essigsäure, Buttersäure) (fermentierende Mikroorganismen) oder eine Methankonzentration (methanogene Mikroorganismen), bestimmt, insbesondere kontinuierlich gemessen, wird (180, 181, 182).

30. Verfahren (100) zur Behandlung von Abwässern nach mindestens dem voranstehenden Anspruch,
**dadurch gekennzeichnet, dass** bei dem unter Verwendung der bestimmten Konzentration der von den Mikroorganismen (44) erzeugten Abbauprodukte und/oder der bestimmten Konzentration der von den mit den Mikroorganismen (44) konkurrierenden (Mikro-)Organismen erzeugten Abbauprodukte eine Behandlungsumgebung des Abwassers (10) im Reaktorraum (2), insbesondere Biofilm (44), kontrolliert wird (185, 184).

31. Verfahren (100) zur Behandlung von Abwässern nach mindestens einem der voranstehenden Ansprüche,
**dadurch gekennzeichnet, dass** ein Verhältnis zwischen den Biofilm (44) erzeugenden Mikroorganismen (44) und den mit den den Biofilm (44) erzeugenden Mikroorganismen (44) konkurrierenden (Mikro-)Organismen überwacht wird (185, 184), insbesondere gesteuert und/oder geregelt wird, insbesondere durch Regulierung einer Aufenthaltsdauer des Abwassers (10) im Reaktorraum (2), durch Regulierung des pH-Werts des Abwassers (10) im Reaktorraum (2), durch eine, insbesondere kurzzeitigen, Sauerstoffdosierung im Reaktorraum (2) und/oder durch eine Steuerung eines Verhältnisses der Substratkonzentration zur Sulfatkonzentration im Abwasser (10).

32. Verfahren (100) zur Behandlung von Abwässern nach mindestens einem der voranstehenden Ansprüche,
**dadurch gekennzeichnet, dass** die Vorrichtung zur Behandlung von Abwässern eine Vorrichtung (1) gemäß einem der voranstehenden Vorrichtungsansprüche ist.

33. Verfahren (100) zur Behandlung von Abwässern nach mindestens einem der voranstehenden Ansprüche,
eingesetzt zu einer Behandlung, insbesondere Reinigung, des Abwassers (10), wobei das Abwasser (10) mit dem auf dem Trägermaterial (41) immobilisierten Biofilm (44) in Kontakt gebracht wird (110) und dabei Fällungsprodukte (43), insbesondere Metallsulfide, aus dem Abwasser (10) ausgefällt und die Fällungsprodukte (43), insbesondere die ausgefällten Metallsulfide, bei der Umwälzung abgeschieden werden (120), wodurch das Abwasser (10) behandelt bzw. gereinigt wird.

34. Verfahren (100) zur Behandlung von Abwässern nach mindestens einem der voranstehenden Ansprüche,
eingesetzt zu einer, insbesondere periodischen, Erneuerung des Biofilms (44), wobei die Erneuerung des Biofilms (44) über die Umwälzung reguliert wird, insbesondere durch eine über die Umwälzung regulierte Biobettwanderungsgeschwindigkeit.

35. Verfahren (100) zur Behandlung von Abwässern, insbesondere von sulfat- und/oder schwermetallhaltigen Minenwässern, bei dem bei einem Abwasser (10), insbesondere bei einem sulfat- und/oder schwermetallhaltigen Minenwasser (10), zunächst ein Membranverfahren (170), insbesondere eine Nanofiltration (170), und anschließend bei einem bei dem Membranverfahren (170), insbesondere bei der Nanofiltration (170), erzeugten Konzentrat (174) eine biologische Sulfatreduktion (172) durchgeführt wird.

36. Verfahren (100) zur Behandlung von Abwässern nach mindestens dem voranstehenden Anspruch,
**dadurch gekennzeichnet, dass** die biologische Sulfatreduktion (171) unter Verwendung einer Vorrichtung (1) gemäß mindestens einem der voranstehenden Vorrichtungsansprüche und/oder nach einem Verfahren (100) gemäß mindestens einem der voranstehenden Verfahrensansprüche durchgeführt wird.
